(19) Europäisches Patentamt
European Patent Office
Office européen des brevets

(11) EP 4 582 416 A1

(12) **EUROPEAN PATENT APPLICATION**
published in accordance with Art. 153(4) EPC

(43) Date of publication:
**09.07.2025 Bulletin 2025/28**

(21) Application number: **23859307.3**

(22) Date of filing: **28.08.2023**

(51) International Patent Classification (IPC):
***C07D 401/02*** (2006.01) ***C07D 401/06*** (2006.01)
***C07D 471/08*** (2006.01) ***C07D 451/06*** (2006.01)
***C07D 495/04*** (2006.01) ***C07D 491/06*** (2006.01)
***C07D 471/04*** (2006.01) ***A61K 31/439*** (2006.01)
***A61K 31/706*** (2006.01) ***A61P 9/00*** (2006.01)
***A61P 13/12*** (2006.01) ***A61P 25/00*** (2006.01)
***A61P 27/02*** (2006.01)

(52) Cooperative Patent Classification (CPC):
**A61K 31/439; A61K 31/706; A61P 9/00; A61P 13/12; A61P 25/00; A61P 27/02; C07D 401/02; C07D 401/06; C07D 451/06; C07D 471/04; C07D 471/08; C07D 491/06; C07D 495/04**

(86) International application number:
**PCT/CN2023/115288**

(87) International publication number:
**WO 2024/046277 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.08.2022 CN 202211064029**
**09.10.2022 CN 202211231001**
**22.08.2023 CN 202311066347**

(71) Applicant: **CMS Research & Development Pte. Ltd.**
**179803 Singapore (SG)**

(72) Inventors:
- **YAN, Xiaobing**
**Shanghai 200131 (CN)**
- **QIAN, Wenyuan**
**Shanghai 200131 (CN)**
- **CHEN, Shuhui**
**Shanghai 200131 (CN)**

(74) Representative: **Haseltine Lake Kempner LLP**
**One Portwall Square**
**Portwall Lane**
**Bristol BS1 6BH (GB)**

(54) **SERIES OF NITROGEN-CONTAINING BRIDGED HETEROCYCLIC COMPOUNDS AND PREPARATION METHOD THEREFOR**

(57) Disclosed in the present invention are a series of nitrogen-containing bridged heterocyclic compounds and a preparation method therefor, and particularly a compound represented by formula (I) and a pharmaceutically acceptable salt thereof. In particular, the present invention also relates to a preparation method for the compound, a pharmaceutical composition, and a use thereof in treating inflammatory diseases.

EP 4 582 416 A1

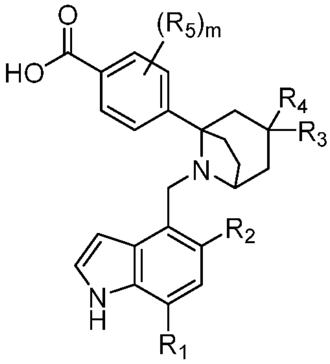

( I )

## Description

CROSS-REFERENCE TO RELATED APPLICATIONS

The present application claims the priority and rights of Chinese invention patent application No. 2022110640297 filed with the China National Intellectual Property Administration on August 29, 2022, Chinese invention patent application No. 2022112310018 filed with the China National Intellectual Property Administration on October 9, 2022, and Chinese invention patent application No. 2023110663471 filed with the China National Intellectual Property Administration on August 22, 2023. The entire contents disclosed in the applications are incorporated herein by reference in their entireties.

TECHNICAL FIELD

The present disclosure relates to a series of nitrogen-containing bridged heterocyclic compounds and a preparation method therefor, specifically to a compound of formula (I) and a pharmaceutically acceptable salt thereof.

BACKGROUND

The complement system consists of more than 50 kinds of proteins that can be precisely regulated, which is an important part of human innate immunity and a bridge between innate immunity and acquired immunity. The complement system is mainly activated through three pathways: the classical pathway (CP), the lectin pathway (LP) and the alternative pathway (AP).

These three activation pathways are all centered on the formation of C3 convertase and C5 convertase. By cleavage of C3 and C5, corresponding bioactive fragments are generated to further amplify the complement reaction. These fragments modify the target surface and can promote processes such as phagocytosis, inflammation and immune regulation. Among them, contacting the activation initiator to generating C3 convertase (and cleavage of C3) can be regarded as the front-end reaction of these activation pathways. Although the front-end reactions of these activation pathways are different, they have a common terminal pathway, that is, C5 convertase is generated, which cleaves C5 to form C5b fragment, and reacts sequentially with C6, C7, C8 and C9 to finally form membrane attack complex (MAC) to exert cytolytic effects.

Complement Factor B acts on the AP pathway. Inhibiting the activity of Factor B can prevent the activation of the AP pathway without interfering with the CP and LP pathways, and can reduce the risk of infection caused by the increase of complement system inhibition. There is no small-molecule Factor B inhibitor on the market yet. Factor B inhibitor LNP023 from Novartis is in the phase III clinical research stage for the treatment of PNH, IgAN, C3G, and other diseases. Therefore, it is necessary to develop a novel small molecule inhibitor of complement system Factor B, increase clinical research and verification, and apply it to the treatment of various diseases caused by complement abnormalities, so as to provide a novel treatment method for unmet clinical needs.

CONTENT OF THE PRESENT INVENTION

The present disclosure provides a compound of formula (I) or a pharmaceutically acceptable salt thereof,

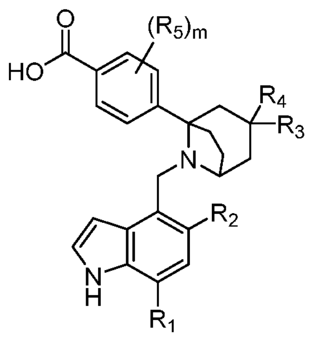

(I)

,

wherein

$R_1$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_a$;

$R_2$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_b$;
$R_3$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_c$;
$R_4$ is selected from $C_{1-6}$ alkylthio, $C_{2-6}$ alkenyl, $C_{2-6}$ alkynyl, $C_{3-6}$ cycloalkyl and 3- to 6-membered heterocyclyl, and the $C_{1-6}$ alkylthio, $C_{2-6}$ alkenyl, $C_{2-6}$ alkynyl, $C_{3-6}$ cycloalkyl and 3- to 6-membered heterocyclyl are each independently and optionally substituted by 1, 2, 3 or 4 $R_d$;
each $R_5$ is independently selected from D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_e$;
each $R_a$ is independently selected from D, F, Cl, Br and I;
each $R_b$ is independently selected from D, F, Cl, Br and I;
each $R_c$ is independently selected from D, F, Cl, Br and I;
each $R_d$ is independently selected from D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 R;
each $R_e$ is independently selected from D, F, Cl, Br and I;
each R is independently selected from D, F, Cl, Br and I;
m is selected from 0, 1, 2 and 3.

The present disclosure further provides a compound of formula (I) or a pharmaceutically acceptable salt thereof,

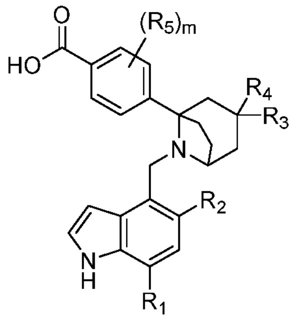

(I)

,

wherein

$R_1$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_a$;
$R_2$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_b$;
$R_3$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_c$;
$R_4$ is selected from $C_{1-6}$ alkylthio, $C_{2-6}$ alkenyl, $C_{2-6}$ alkynyl and $C_{3-6}$ cycloalkyl, and the $C_{1-6}$ alkylthio, $C_{2-6}$ alkenyl, $C_{2-6}$ alkynyl and $C_{3-6}$ cycloalkyl are each independently and optionally substituted by 1, 2, 3 or 4 $R_d$;
each $R_5$ is independently selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_e$;
each $R_a$ is independently selected from D, F, Cl, Br and I;
each $R_b$ is independently selected from D, F, Cl, Br and I;
each $R_c$ is independently selected from D, F, Cl, Br and I;
each $R_d$ is independently selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 R;
each $R_e$ is independently selected from D, F, Cl, Br and I;
each R is independently selected from D, F, Cl, Br and I;
m is selected from 0, 1, 2 and 3.

In some embodiments of the present disclosure, each $R_d$ is independently selected from D, F, Cl, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 R, and other variables are as defined in the present disclosure.

In some embodiments of the present disclosure, each $R_d$ is independently selected from F and $C_{1-3}$ alkyl, and the

$C_{1-3}$ alkyl is optionally substituted by 1, 2 or 3 R, and other variables are as defined in the present disclosure.

In some embodiments of the present disclosure, each $R_d$ is independently selected from F, methyl and ethyl, and the methyl and ethyl are each independently and optionally substituted by 1, 2, or 3 R, and other variables are as defined in the present disclosure.

In some embodiments of the present disclosure, each $R_d$ is independently selected from F and methyl, and other variables are as defined in the present disclosure.

In some embodiments of the present disclosure, each $R_d$ is independently selected from D, F and Cl, and other variables are as defined in the present disclosure.

In some embodiments of the present disclosure, each $R_d$ is independently selected from F, and other variables are as defined in the present disclosure.

In some embodiments of the present disclosure, each $R_d$ is independently selected from H and F, and other variables are as defined in the present disclosure. In some embodiments of the present disclosure, each $R_a$, each $R_b$, each $R_c$, each $R_e$ and each R are independently selected from D and F, and other variables are as defined in the present disclosure.

In some embodiments of the present disclosure, each $R_a$, each $R_b$, each $R_c$, each $R_e$ and each R are independently selected from D, and other variables are as defined in the present disclosure.

In some embodiments of the present disclosure, the $R_1$ is selected from $C_{1-3}$ alkyl, and the $C_{1-3}$ alkyl is optionally substituted by 1, 2 or 3 $R_a$, and $R_a$ and other variables are as defined in the present disclosure.

In some embodiments of the present disclosure, the $R_1$ is selected from $CH_3$, and the $CH_3$ is optionally substituted by 1, 2 or 3 $R_a$, and $R_a$ and other variables are as defined in the present disclosure.

In some embodiments of the present disclosure, the $R_1$ is selected from $CH_3$ and $CD_3$, and other variables are as defined in the present disclosure.

In some embodiments of the present disclosure, the $R_1$ is selected from $CH_3$, and other variables are as defined in the present disclosure.

In some embodiments of the present disclosure, the $R_2$ is selected from $C_{1-3}$ alkoxy, and the $C_{1-3}$ alkoxy is optionally substituted by 1, 2 or 3 $R_b$, and $R_b$ and other variables are as defined in the present disclosure.

In some embodiments of the present disclosure, the $R_2$ is selected from $OCH_3$, and the $OCH_3$ is optionally substituted by 1, 2 or 3 $R_b$, and $R_b$ and other variables are as defined in the present disclosure.

In some embodiments of the present disclosure, the $R_2$ is selected from $OCH_3$ and $OCD_3$, and other variables are as defined in the present disclosure.

In some embodiments of the present disclosure, the $R_2$ is selected from $OCH_3$, and other variables are as defined in the present disclosure.

In some embodiments of the present disclosure, the $R_3$ is selected from H, F and $C_{1-3}$ alkyl, and the $C_{1-3}$ alkyl is optionally substituted by 1, 2 or 3 $R_c$, and other variables are as defined in the present disclosure.

In some embodiments of the present disclosure, the $R_3$ is selected from H, F and $CH_3$, and other variables are as defined in the present disclosure.

In some embodiments of the present disclosure, the $R_3$ is selected from H, and other variables are as defined in the present disclosure.

In some embodiments of the present disclosure, the $R_4$ is selected from $C_{1-6}$ alkylthio, $C_{2-6}$ alkenyl, $C_{2-6}$ alkynyl, $C_{3-6}$ cycloalkyl and 4- to 6-membered heterocycloalkyl, and the $C_{1-6}$ alkylthio, $C_{2-6}$ alkenyl, $C_{2-6}$ alkynyl, $C_{3-6}$ cycloalkyl and 4- to 6-membered heterocycloalkyl are each independently and optionally substituted by 1, 2, 3 or 4 $R_d$, and other variables are as defined in the present disclosure.

In some embodiments of the present disclosure, the $R_4$ is selected from $C_{1-6}$ alkylthio, $C_{2-6}$ alkenyl, $C_{2-6}$ alkynyl and $C_{3-6}$ cycloalkyl, and the $C_{1-6}$ alkylthio, $C_{2-6}$ alkenyl, $C_{2-6}$ alkynyl and $C_{3-6}$ cycloalkyl are each independently and optionally substituted by 1, 2, 3 or 4 $R_d$, and other variables are as defined in the present disclosure.

In some embodiments of the present disclosure, the $R_4$ is selected from $C_{1-3}$ alkylthio, $C_{2-3}$ alkenyl, $C_{2-3}$ alkynyl and $C_{3-4}$ cycloalkyl, and the $C_{1-3}$ alkylthio, $C_{2-3}$ alkenyl, $C_{2-3}$ alkynyl and $C_{3-4}$ cycloalkyl are each independently and optionally substituted by 1, 2, 3 or 4 $R_d$, and $R_d$ and other variables are as defined in the present disclosure.

In some embodiments of the present disclosure, the $R_4$ is selected from

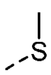, 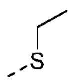, 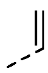,

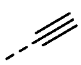 and 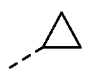, and the 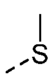, 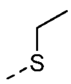, 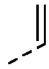, 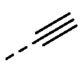 and 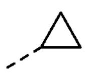

are each independently and optionally substituted by 1, 2, 3 or 4 $R_d$, and $R_d$ and other variables are as defined in the present disclosure.

In some embodiments of the present disclosure, the $R_4$ is selected from

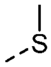, 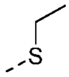, 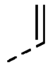,

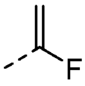, 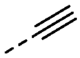 and 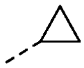,

and other variables are as defined in the present disclosure.

In some embodiments of the present disclosure, the $R_4$ is selected from

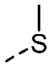, 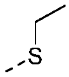, 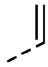,

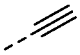, 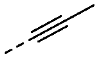, 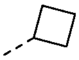 and 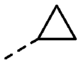, and the 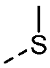, 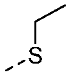, 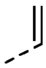, 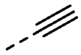, 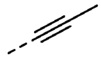, 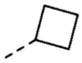

and

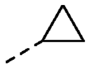

are each independently and optionally substituted by 1, 2, 3 or 4 $R_d$, and $R_d$ and other variables are as defined in the present disclosure.

In some embodiments of the present disclosure, the $R_4$ is selected from

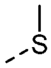, 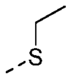, 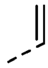,

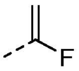, 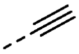, 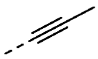, 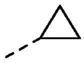, 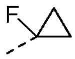, 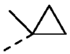, 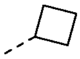 and 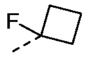,

and other variables are as defined in the present disclosure.

In some embodiments of the present disclosure, the $R_4$ is selected from $C_{1-3}$ alkylthio and $C_{3-6}$ cycloalkyl, and the $C_{1-3}$ alkylthio and $C_{3-6}$ cycloalkyl are each independently and optionally substituted by 1, 2, 3 or 4 $R_d$, and other variables are as defined in the present disclosure.

In some embodiments of the present disclosure, the $R_4$ is selected from $C_{1-3}$ alkylthio and $C_{3-4}$ cycloalkyl, and the $C_{1-3}$ alkylthio and $C_{3-4}$ cycloalkyl are each independently and optionally substituted by 1, 2, 3 or 4 $R_d$, and other variables are as defined in the present disclosure.

In some embodiments of the present disclosure, the $R_4$ is selected from $C_{3-4}$ cycloalkyl, and the $C_{3-4}$ cycloalkyl is optionally substituted by 1, 2, 3 or 4 $R_d$, and other variables are as defined in the present disclosure.

In some embodiments of the present disclosure the $R_4$ is selected from

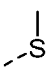, 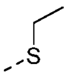, 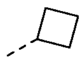

and

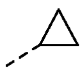, and the 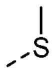, 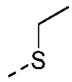, 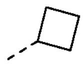 and 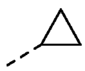

are each independently and optionally substituted by 1, 2, 3 or 4 $R_d$, and $R_d$ and other variables are as defined in the present disclosure.

In some embodiments of the present disclosure, the $R_4$ is selected from

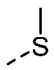, 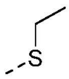, 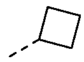

and

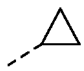

, and other variables are as defined in the present disclosure.

In some embodiments of the present disclosure, the $R_4$ is selected from

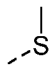, 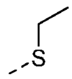

and

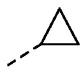,

and other variables are as defined in the present disclosure.

In some embodiments of the present disclosure, the $R_4$ is selected from

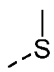 and 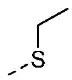,

and other variables are as defined in the present disclosure.

In some embodiments of the present disclosure, the $R_4$ is selected from

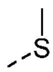 and 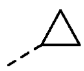,

and other variables are as defined in the present disclosure.

In some embodiments of the present disclosure, the $R_4$ is selected from variables are as defined in the present disclosure.

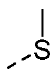,

and other

In some embodiments of the present disclosure, each $R_5$ is independently selected from F or $C_{1-3}$ alkyl, and the $C_{1-3}$ alkyl is optionally substituted by 1, 2 or 3 $R_e$, and other variables are as defined in the present disclosure.

In some embodiments of the present disclosure, each $R_5$ is independently selected from F, and other variables are as defined in the present disclosure.

In some embodiments of the present disclosure, each $R_5$ is independently selected from H, and other variables

are as defined in the present disclosure.

In some embodiments of the present disclosure, the m is selected from 0, and other variables are as defined in the present disclosure.

In some embodiments of the present disclosure, the compound of formula (I) or the pharmaceutically acceptable salt thereof is selected from a compound of formula (P),

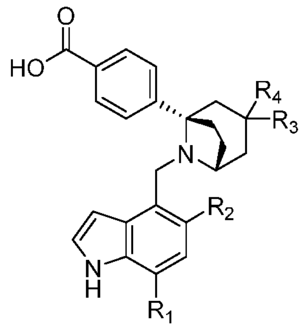

(P)

,

wherein $R_1$, $R_2$, $R_3$ and $R_4$ are as defined in the present disclosure.

In some embodiments of the present disclosure, the compound of formula (I) or formula (P) or the pharmaceutically acceptable salt thereof is selected from a compound of formula (P-1) or a compound of formula (P-2),

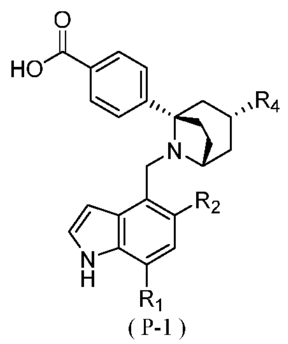

(P-1)

and

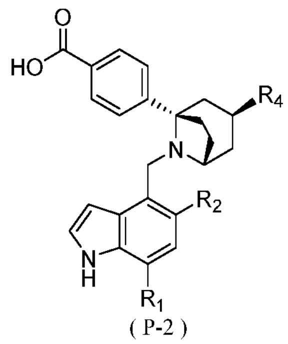

(P-2)

,

wherein $R_1$, $R_2$ and $R_4$ are as defined in the present disclosure.

In some embodiments of the present disclosure, the compound of formula (I) or the pharmaceutically acceptable salt thereof is selected from a compound of formula (P), a compound of formula (P-1) or a compound of formula (P-2), wherein

$R_1$ is selected from $C_{1-3}$ alkyl, and the $C_{1-3}$ alkyl is optionally substituted by 1, 2 or 3 $R_a$;
$R_2$ is selected from $C_{1-3}$ alkoxy, and the $C_{1-3}$ alkoxy is optionally substituted by 1, 2 or 3 $R_b$;
$R_3$ is selected from H;
$R_4$ is selected from $C_{1-3}$ alkylthio, $C_{2-3}$ alkenyl, $C_{2-3}$ alkynyl and $C_{3-4}$ cycloalkyl, and the $C_{1-3}$ alkylthio, $C_{2-3}$ alkenyl, $C_{2-3}$ alkynyl and $C_{3-4}$ cycloalkyl are each independently and optionally substituted by 1, 2, 3 or 4 $R_d$;
each $R_a$ is independently selected from D, F, Cl, Br and I;
each $R_b$ is independently selected from D, F, Cl, Br and I;
each $R_d$ is independently selected from D, F, Cl, Br, I, $C_{1-3}$ alkyl and $C_{1-3}$ alkoxy, and the $C_{1-3}$ alkyl and $C_{1-3}$ alkoxy are each independently and optionally substituted by 1, 2 or 3 R;
each R is independently selected from D, F, Cl, Br and I.

In some embodiments of the present disclosure, $R_4$ in the compound of formula (P-1) or the compound of formula (P-2) is as defined in the compound of formula (I) or the pharmaceutically acceptable salt thereof.

There are still some embodiments of the present disclosure which are obtained by any combination of the above variables.

The present disclosure further provides a compound as shown below or a pharmaceutically acceptable salt thereof,

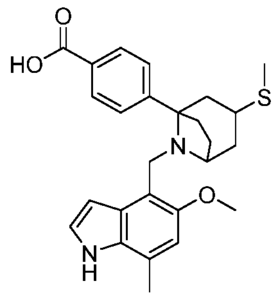, 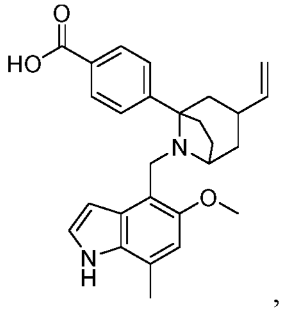, 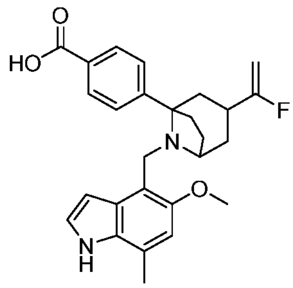, 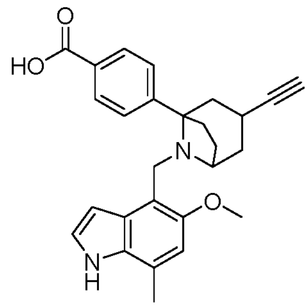,

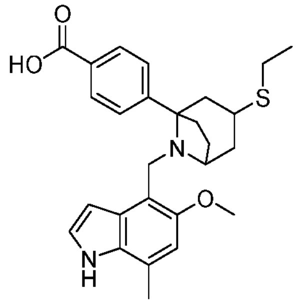, 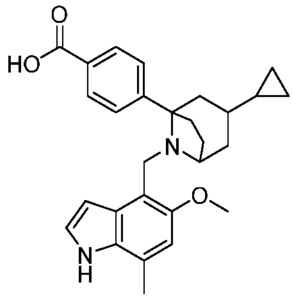, 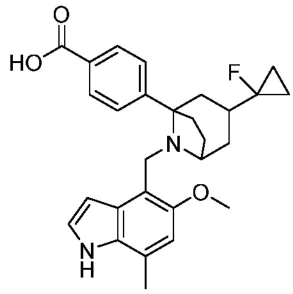, 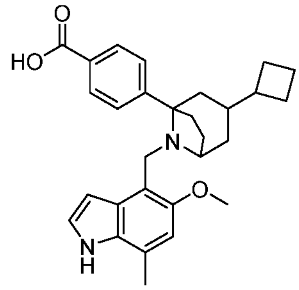,

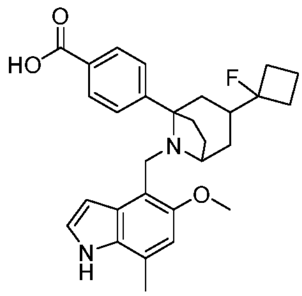, 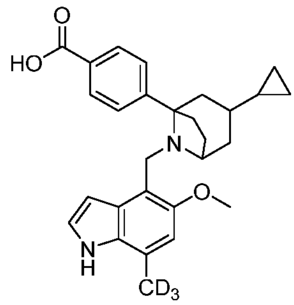, 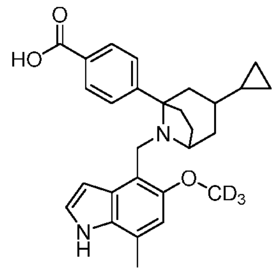, 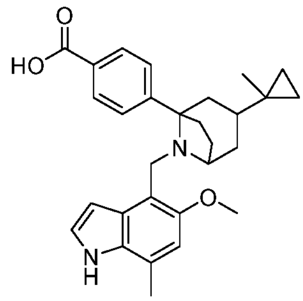,

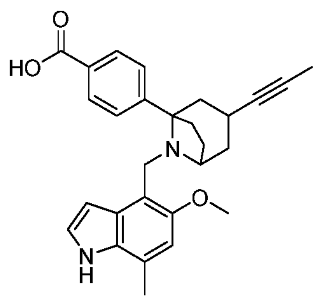, 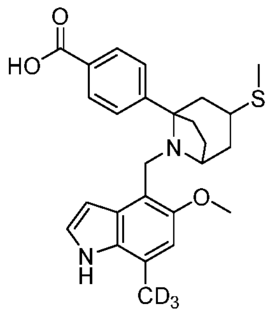, 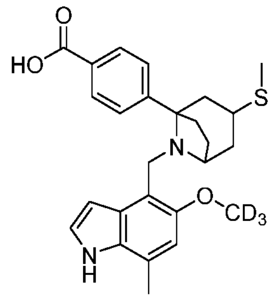 and 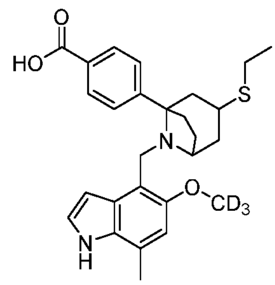.

In some embodiments of the present disclosure, the compound or the pharmaceutically acceptable salt thereof is selected from:

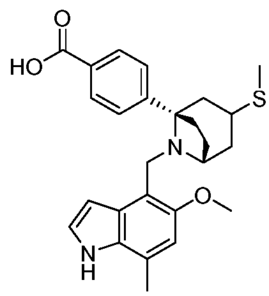 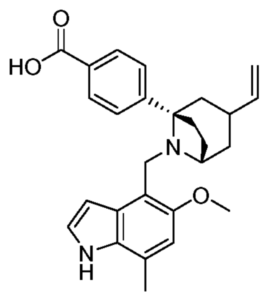 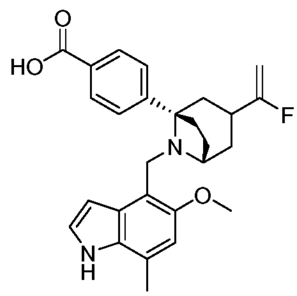 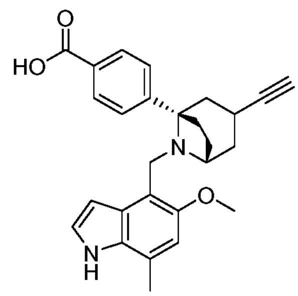

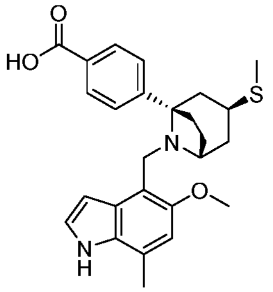
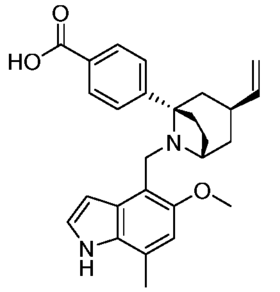
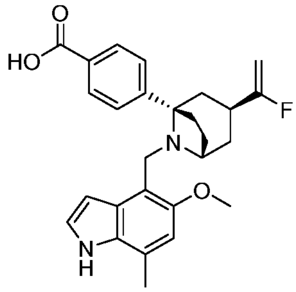
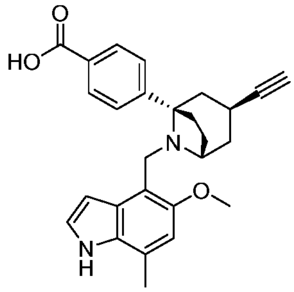

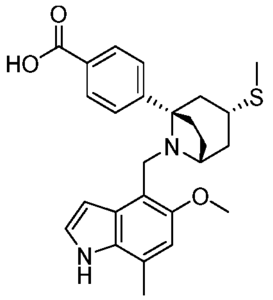
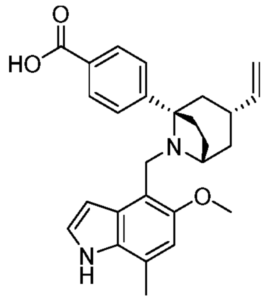
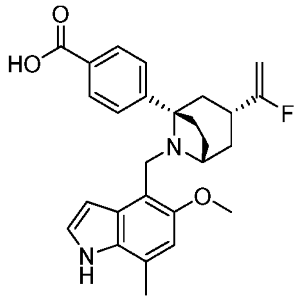
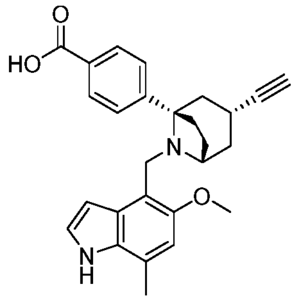

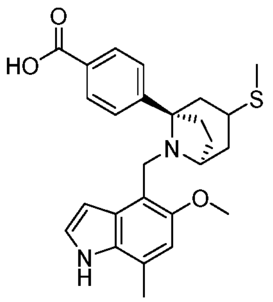
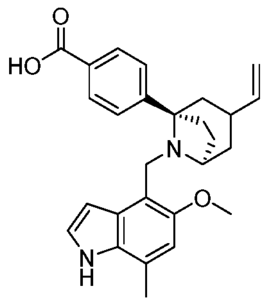
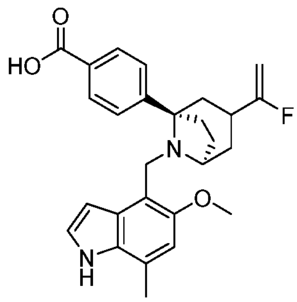
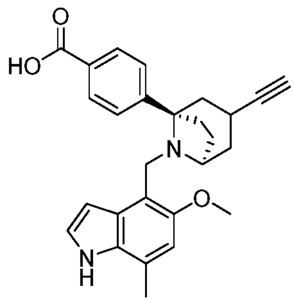

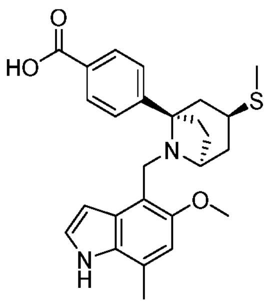
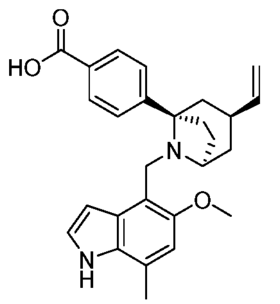
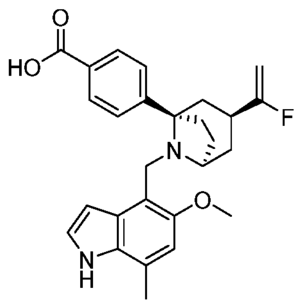
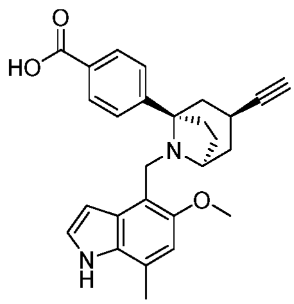

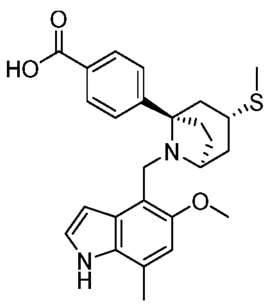
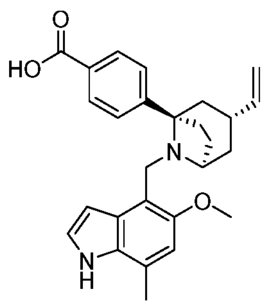
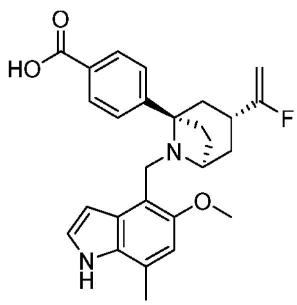
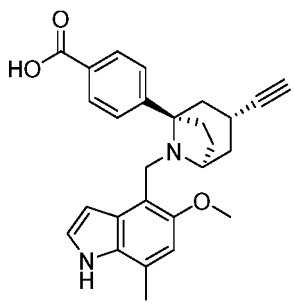

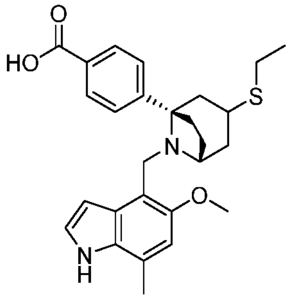
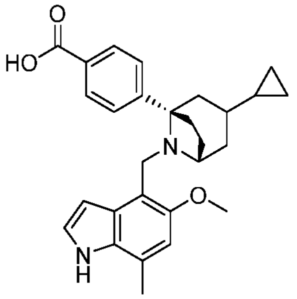
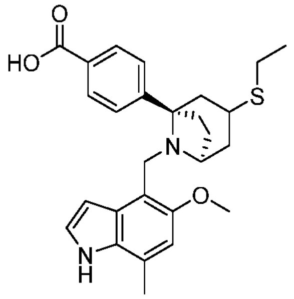
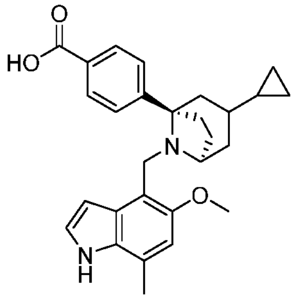

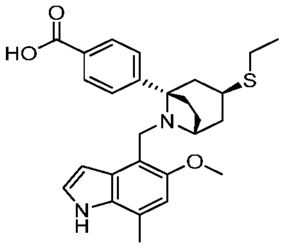
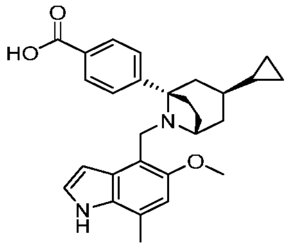
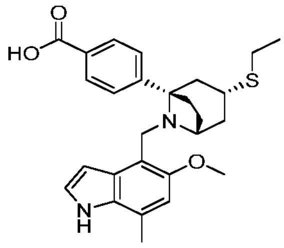
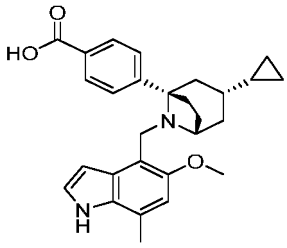

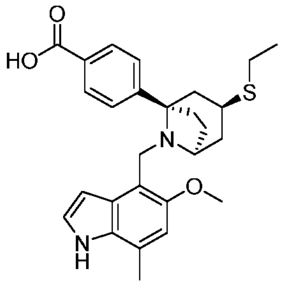
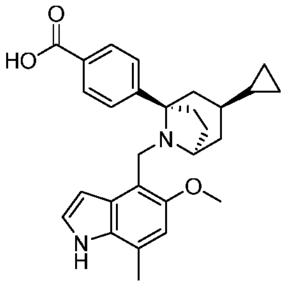
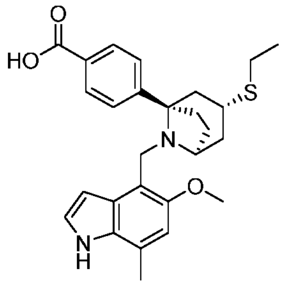
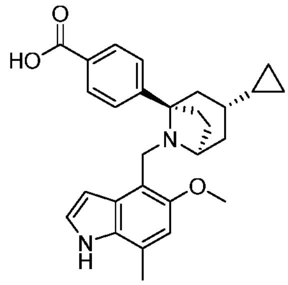

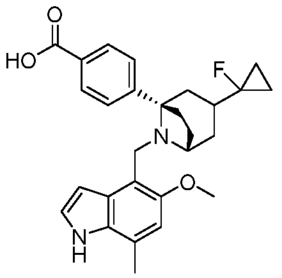
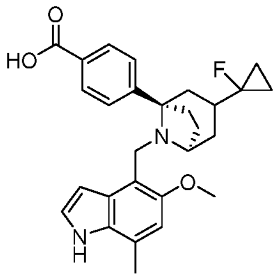
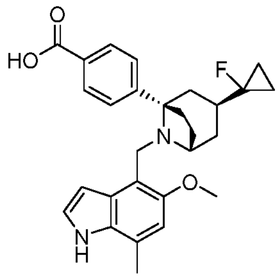
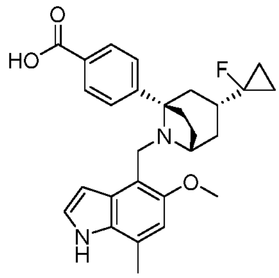

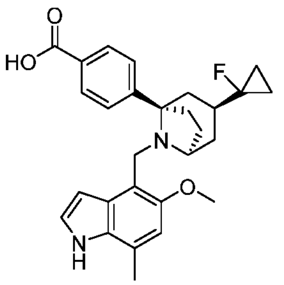
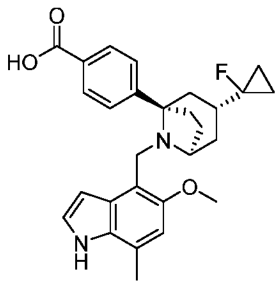
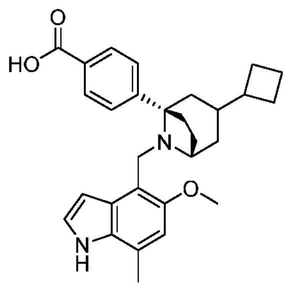
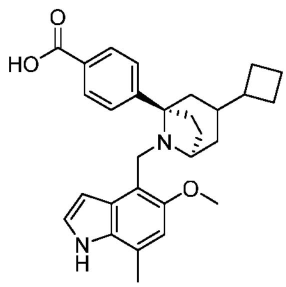

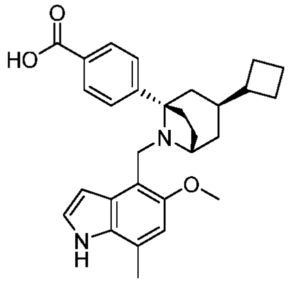
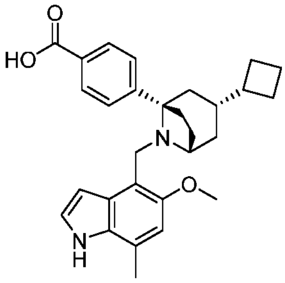
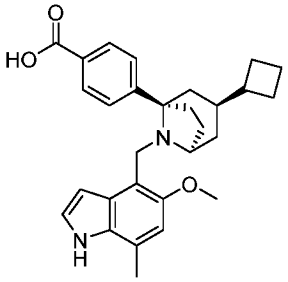
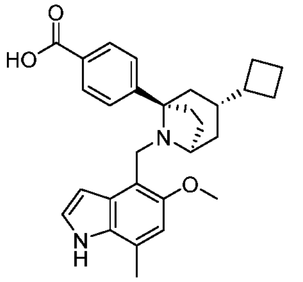

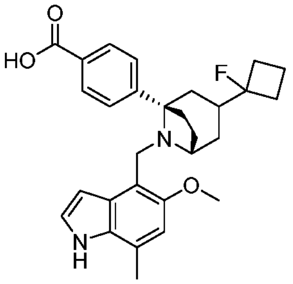
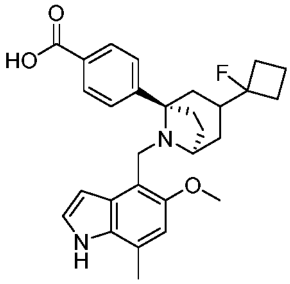
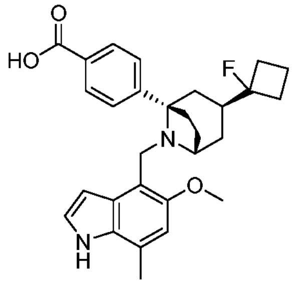
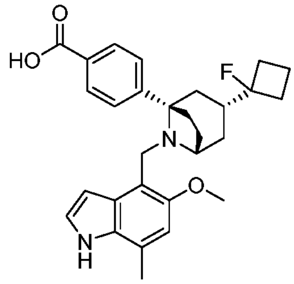

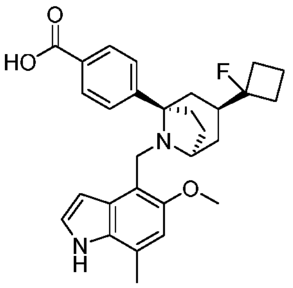
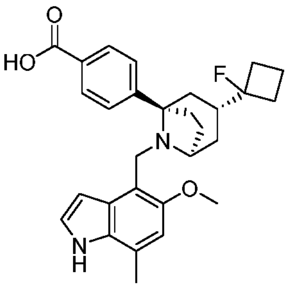
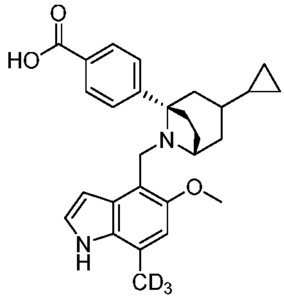
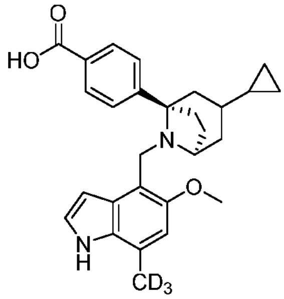

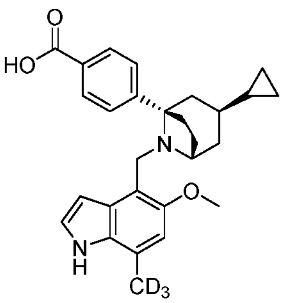
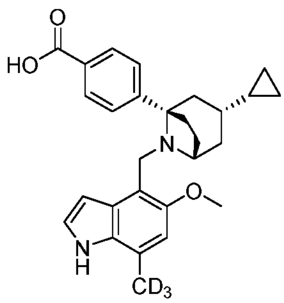
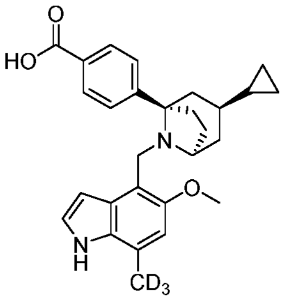
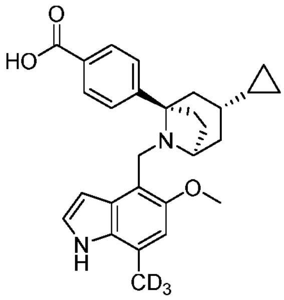

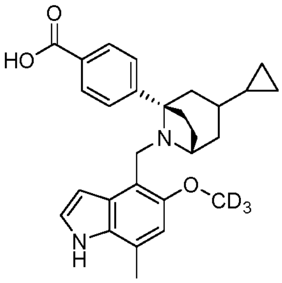
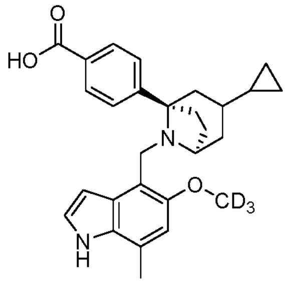
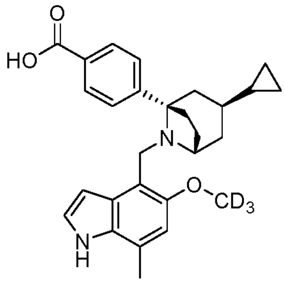
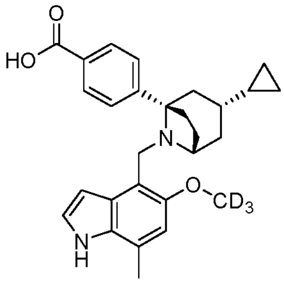

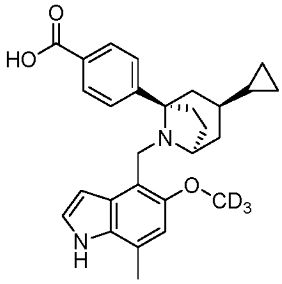
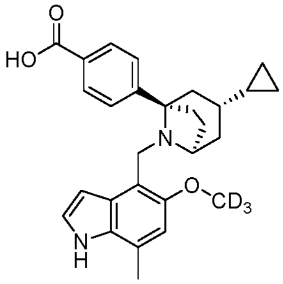
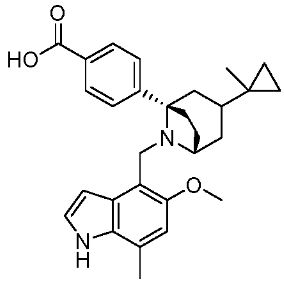
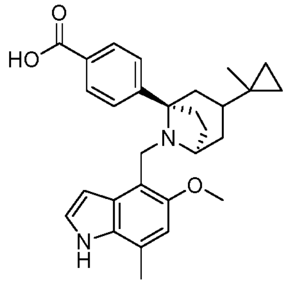
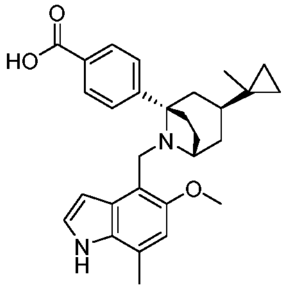
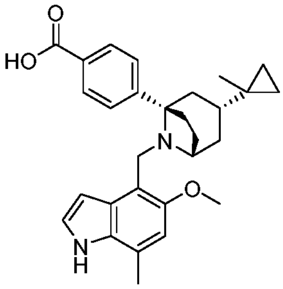
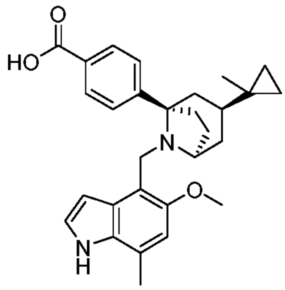
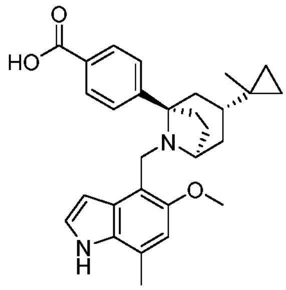
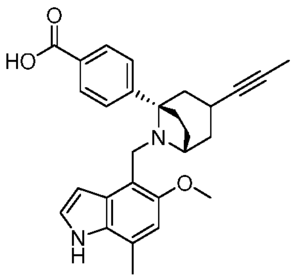
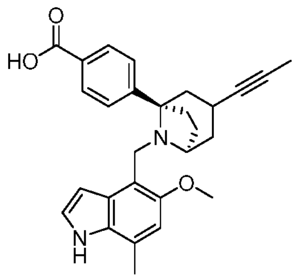
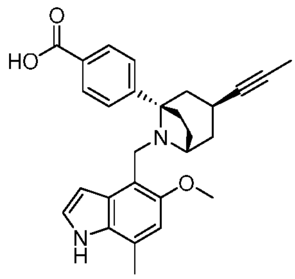
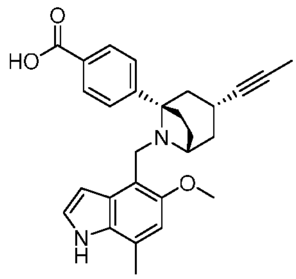
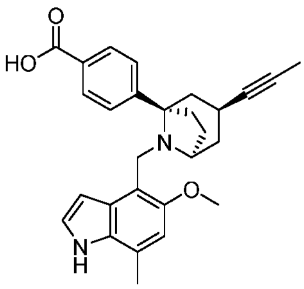
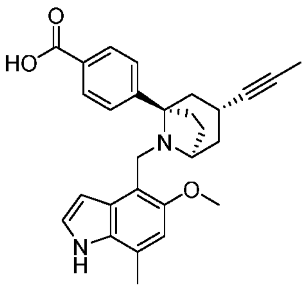
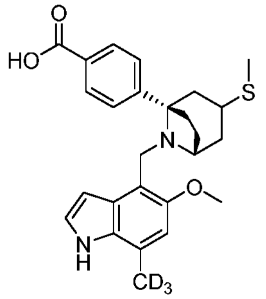
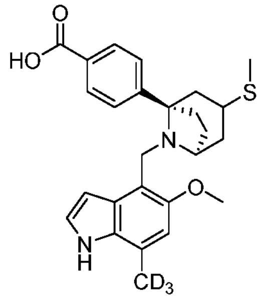
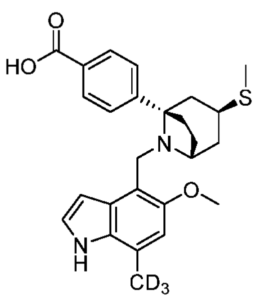
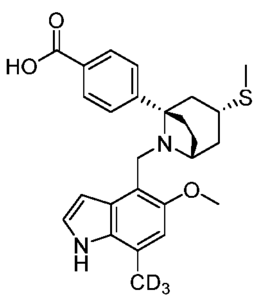
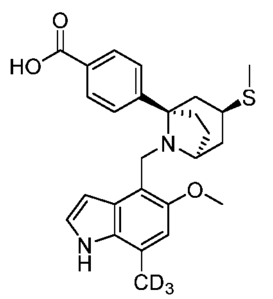
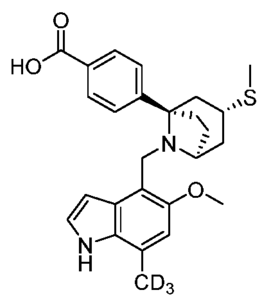

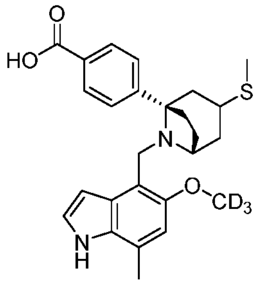 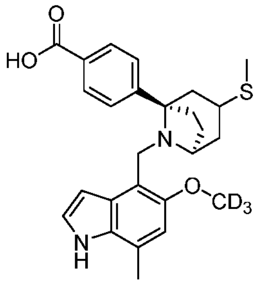 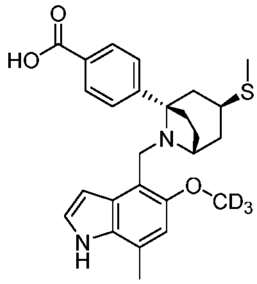 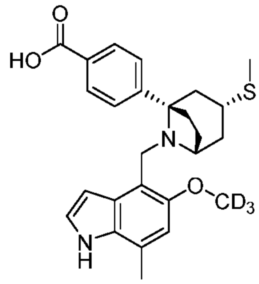

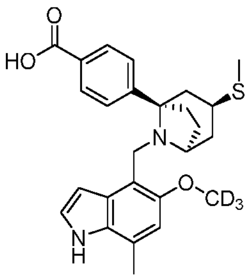 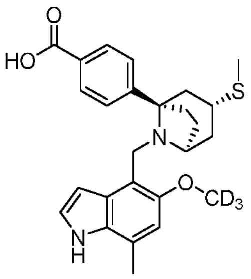 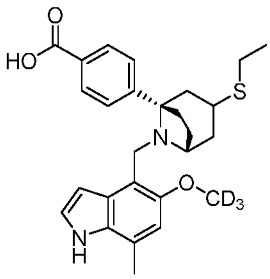 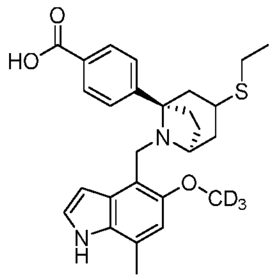

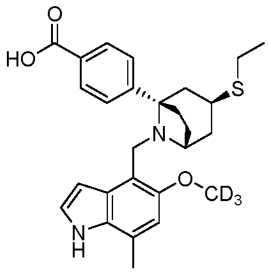 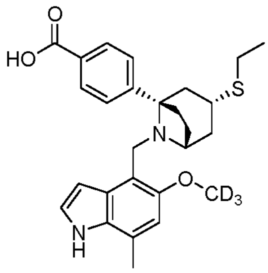 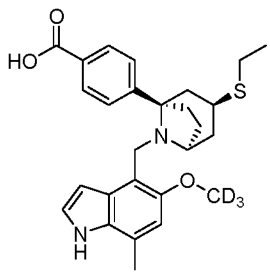 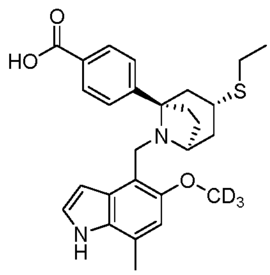 .

The present disclosure further provides a pharmaceutical composition comprising a therapeutically effective amount of the compound or the pharmaceutically acceptable salt thereof of the present disclosure. Further, a pharmaceutically acceptable carrier is further included.

The present disclosure further provides a use of the compound or the pharmaceutically acceptable salt thereof in the manufacture of a medicament for treating diseases associated with complement Factor B.

The present disclosure further provides a method for treating diseases associated with complement Factor B, comprising administering a therapeutically effective amount of the compound or the pharmaceutically acceptable salt thereof of the present disclosure to a mammal (preferably a human) in need of the treatment.

The present disclosure further provides a use of the compound or the pharmaceutically acceptable salt thereof in treating diseases associated with complement Factor B.

The present disclosure further provides the compound or the pharmaceutically acceptable salt thereof for use in treating diseases associated with complement Factor B.

In some embodiments of the present disclosure, the diseases associated with complement Factor B are selected from inflammatory disorders and autoimmune diseases.

In some embodiments of the present disclosure, the pharmaceutically acceptable salt is selected from a pharmaceutically acceptable acid addition salt.

In some embodiments of the present disclosure, the pharmaceutically acceptable salt is selected from a pharmaceutically acceptable inorganic acid salt, organic acid salt and amino acid salt.

In some embodiments of the present disclosure, the pharmaceutically acceptable salt is selected from a formate salt.

Stereoisomers and/or tautomers of the compounds of formula (I) or pharmaceutically acceptable salts thereof of the present disclosure are further included in the scope of the present disclosure.

The present disclosure further provides a synthesis method for the compound, and the synthesis route is as follows:

Synthetic route 1:

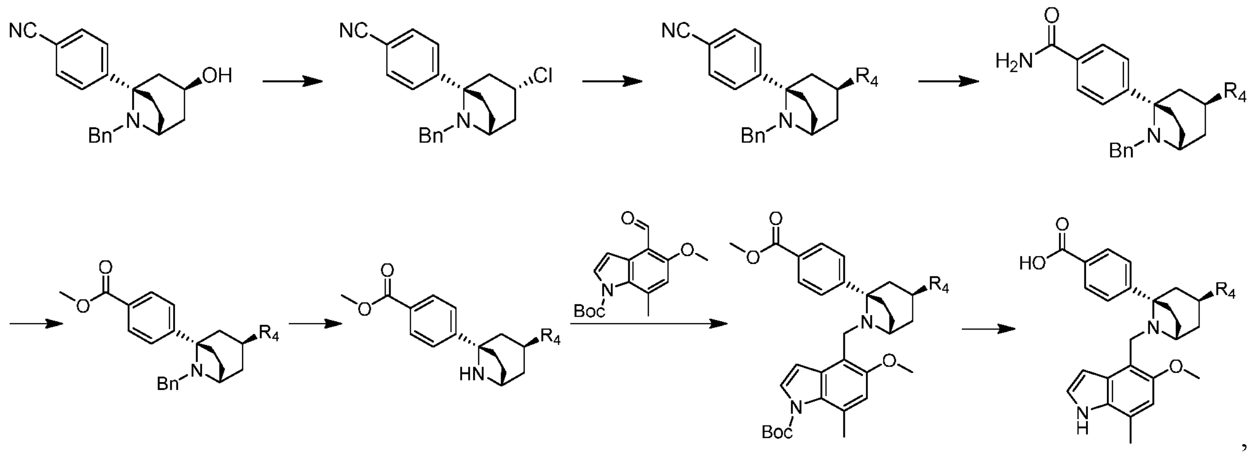

,

wherein $R_4$ is as defined above.

In some embodiments of the present disclosure, $R_4$ is selected from

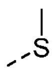 , 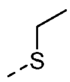 , 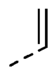 , 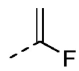

and

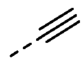 .

The present disclosure further provides biological test methods for the compounds:

**Experimental Test Method 1: Wieslab Complement Alternative Pathway Activation Inhibition (Enzyme Activity Test)**

Experimental purpose:

To determine the inhibitory activity of the test compounds on the complement alternative pathway in human serum by using the Wieslab® complement system alternative pathway kit.

Experimental scheme:

Serum is diluted with diluent (1:23). Drugs are added to the diluted serum with 8 concentration gradients of up to 10 mM or 50 mM in a 5-fold serial dilution. The mixture is incubated at room temperature for 15 min. The mixture of compound and serum is added to a 96-well plate (100 μL/well) provided in the kit, and activated at 37°C for 1 hour. The plate is washed three times with wash buffer. The plate is added with detection antibody (100 μL) provided in the kit and incubated at room temperature for 30 min. The plate is washed three times with wash buffer. The plate is added with substrate (100 μL) and incubated at room temperature for 30 min. The absorbance is detected at 405 nM on a microplate reader.

Conclusion: The compounds of the present disclosure have significant inhibitory activity on the activation of the alternative pathway in human serum.

**Experimental Test Method 2: Complement Human Factor B Protein Binding Test (TR-FRET Method)**

Experimental purpose:

To determine the binding activity of the test compounds against human complement Factor B protein using the TR-FRET method.

Experimental scheme:

The drug is added to phosphate buffered saline PBS (pH 7.4), and diluted 4 times from 10 μM for a total of 10 concentrations. Biotinylated Factor B (25 nM) is incubated for 2 hours at room temperature after the addition of Cy5-labeled probe (75 nM) and europium chelate-labeled streptavidin (Perkin Elmer #AD0060; 0.225 nM) in the absence or presence of various concentrations of test compounds. Time-resolved fluorescence resonance energy transfer (TR-FRET) data are measured using 330 nm as the excitation wavelength and 665 nm as the emission wavelength.
Conclusion: The compounds of the present disclosure have significant binding activity to human complement Factor B protein.

**Experimental Test Method 3: Pharmacokinetic Study in Rats**

Experimental purpose:

Male SD rats are used as test animals, and the LC/MS/MS method is used to determine the drug concentration in the plasma of the rats at different times after oral gavage administration of the compounds of the present disclosure. The pharmacokinetic behavior of the compounds of the present disclosure in rats *in vivo* is studied to evaluate their pharmacokinetic properties.

Experimental scheme:

Healthy male rats, 200 to 300 g, 2 rats per group.

The compounds of the present disclosure are prepared into a clear solution of 1 mg/mL using a 0.5% MC (4000 cP)/0.5% Tween 80 aqueous solution. Rats are administered after overnight fasting, dose: 10 mg/kg, mode of administration: oral gavage. Blood is collected before and 0.25, 0.5, 1, 2, 4, 7 and 24 hours after administration, placed in heparinized anticoagulant tubes, centrifuged at 7000 rpm (5204 g) and 4°C, and plasma is separated and stored at -80°C. Rats are fed 4 hours after administration. The LC/MS/MS method is used to determine the content of the test compounds in rat plasma after oral administration. Plasma samples are analyzed after pretreatment with precipitated proteins.
Conclusion: The compounds of the present disclosure have good oral exposure and a long half-life, and excellent pharmacokinetic properties.

**Experimental Test Method 4: LPS-Induced Complement Activation *In Vivo* PD Model in Mice**

Experimental purpose:

To investigate the inhibitory effect of the compounds of examples of the present disclosure on complement activation in mice induced by LPS stimulation.

Experimental animals:

Female C57BL/6J mice, 7 to 9 weeks old, weighing 17 to 23 g; supplier: Shanghai Sipple-Bikai Laboratory Animal Co., Ltd.

Experimental procedure:

4 hours before administration, 100 μg of lipopolysaccharide (LPS) in *Salmonella typhimurium* (Sigma) dissolved in 100 μL of sterile PBS (phosphate buffered saline, pH 7.2 to 7.4) is intraperitoneally injected to induce complement activation in mice. Normal mouse group (negative control): Animals are intraperitoneally injected with 100 μL of sterile PBS, and vehicle alone is administered by gavage (PO). Model group (positive control): Animals are intraperitoneally injected with LPS and vehicle is administered by PO (20% PEG400/10% solutol/70% water). Drug group: Sample collection is performed 4 hours after administration of compound (vehicle: 20% PEG400/10% solutol/70% water, administration volume: 5 mL/kg, dose: 10 mg/kg).

Sample collection: 0.3 mL of blood samples are collected from the orbital venous plexus. All blood samples are added to 1.5 mL commercial EDTA-K2 anticoagulant tubes (supplied by Jiangsu Kangjian Medical Apparatus Co., Ltd.). After collection, within half an hour, the blood sample is centrifuged at 4°C, 3000 g for 10 min to aspirate the supernatant plasma, which is put in dry ice immediately, and stored in a refrigerator at -80°C for Western blot analysis of downstream C3d protein levels after complement activation.

Sample analysis: Mouse plasma (5 μL) + Lysis buffer (27.5 μL) + Loading buffer (12.5 μL) + Reducing buffer (5 μL) are mixed well and incubated at 100°C for 15 min with a loading volume of 5 μL/well, i.e., a loading volume of plasma of 0.5 μL per well.

Experimental conclusion: Compared with the normal group and model group, the compounds of the present disclosure can significantly inhibit complement activation stimulated by LPS.

**Technical Effect**

The compounds of the present disclosure have obvious inhibitory activity on the activation of human serum alternative pathway, and also have significant binding activity against human complement Factor B protein. The compounds of the present disclosure have a moderate plasma protein binding rate in the plasma of various species, indicating that in the plasma of various species, the free drug concentration ratio of the compound of the present disclosure is moderate. The compounds f the present disclosure have good pharmacokinetic properties (such as AUC or F) and have good liver microsomal metabolic stability. The compounds have good permeability, can significantly inhibit complement activation stimulated by LPS, and can be developed into a new complement system Factor B small molecule inhibitor.

**Related Definitions**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (R)- and (S)-enantiomers, diastereoisomers, (D)-isomers, (L)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid

bond ( ) and a wedged dashed bond ( ), and the relative configuration of a stereogenic center is represented by a straight solid bond ( ) and a straight dashed bond ( ), a wave line ( ) is used to represent a wedged solid bond ( ) or a wedged dashed bond ( ), or the wave line ( ) is used to represent a straight solid bond ( ) or a straight dashed bond ( ).

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one. Isomers and their mixtures are included within the scope of the present disclosure.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

The compound of the present disclosure may contain an unnatural proportion of atomic isotope at one or more than one atom that constitutes the compound. For example, the compound can be radiolabeled with a radioactive isotope, such as tritium ($^{3}H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (*i.e.*, =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted with up to two R, wherein the definition of R at each occurrence is independent. For example, if a group is substituted by 0 R, it means that the group is unsubstituted. Moreover, a combination of the substituent and/or the variant thereof is allowed only when the combination results in a stable compound.

When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond ( ), a straight dashed bond ( ) or a wavy line

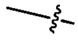 ).

For example, the straight solid bond in $-OCH_3$ means that it is linked to other groups through the oxygen atom in the group; the straight dashed bond in

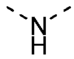

means that it is linked to other groups through the two ends of the nitrogen atom in the group; the wave lines in

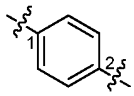

means that the phenyl group is linked to other groups through carbon atoms at position 1 and position 2;

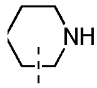

means that it can be linked to other groups through any linkable sites on the piperidinyl by one chemical bond, including at least four types of linkage, including

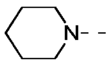 ,

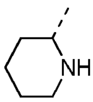 , 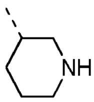 , 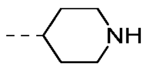 .

Even though the H atom is drawn on the -N-,

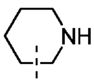

still includes the linkage of

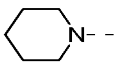 ,

merely when one chemical bond is connected, the H of this site will be reduced by one to the corresponding monovalent piperidinyl.

When a variable (e.g., R) is substituted on one ring of a polycyclic system, unless otherwise specified, the variable (e.g., R) is designated to be substituted on this ring, but cannot be substituted on other rings of the polycyclic system. For example,

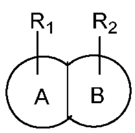

means that ring A and ring B are fused, and the substituent $R_1$ is the substituent of ring A and the substituent $R_2$ is the substituent of ring B, and

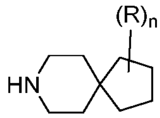

indicates that the five-membered ring in the spirocyclic system is substituted by n R.

Unless otherwise specified, the term "$C_{1-3}$ alkyl" refers to a linear or branched saturated hydrocarbon group consisting of 1 to 3 carbon atoms. The $C_{1-6}$ alkyl includes $C_{1-2}$, $C_{2-3}$ alkyl, *etc.;* it can be monovalent (such as methyl), divalent (such as methylene) or multivalent (such as methine). Examples of $C_{1-3}$ alkyl include, but are not limited to, methyl (Me), ethyl (Et), propyl (including n-propyl and isopropyl), *etc.*

Unless otherwise specified, the term "$C_{1-3}$ alkoxy" refers to an alkyl group containing 1 to 3 carbon atoms that are connected to the rest of the molecule through an oxygen atom. The $C_{1-3}$ alkoxy includes $C_{1-2}$, $C_{2-3}$, $C_3$, $C_2$ alkoxy, *etc.* Examples of $C_{1-3}$ alkoxy include, but are not limited to, methoxy, ethoxy, propoxy (including n-propoxy and isopropoxy), *etc.*

Unless otherwise specified, the term "$C_{1-6}$ alkylthio" refers to an alkyl group containing 1 to 6 carbon atoms that are connected to the rest of the molecule through a sulfur atom. The $C_{1-6}$ alkylthio includes $C_{1-4}$, $C_{1-3}$, $C_{1-2}$, $C_{2-6}$, $C_{2-4}$, $C_6$, $C_5$, $C_4$, $C_3$, $C_2$ alkylthio, *etc.* Examples of $C_{1-6}$ alkylthio include, but are not limited to $-SCH_3$, $-SCH_2CH_3$, $-SCH_2CH_2CH_3$, $-SCH_2(CH_3)_2$, *etc.*

Unless otherwise specified, the term "$C_{1-3}$ alkylthio" refers to an alkyl group containing 1 to 3 carbon atoms that are connected to the rest of the molecule through a sulfur atom. The $C_{1-3}$ alkylthio includes $C_{1-3}$, $C_{1-2}$, $C_3$ alkylthio, *etc.* Examples of $C_{1-3}$ alkylthio include, but are not limited to $-SCH_3$, $-SCH_2CH_3$, $-SCH_2CH_2CH_3$, $-SCH_2(CH_3)_2$, *etc.*

Unless otherwise specified, "$C_{2-6}$ alkenyl" is used to mean a linear or branched hydrocarbon group consisting of 2 to 6 carbon atoms containing at least one carbon-carbon double bond, wherein the carbon-carbon double bond can be located at any position within the group. The $C_{2-6}$ alkenyl includes $C_{2-4}$, $C_{2-3}$, $C_4$, $C_3$, $C_2$ alkenyl, *etc.;* it can be monovalent, divalent or multivalent. Examples of $C_{2-6}$ alkenyl include, but are not limited to, vinyl, propenyl, butenyl, pentenyl, hexenyl, butadienyl, pentadienyl, hexadienyl, *etc.*

Unless otherwise specified, "$C_{2-3}$ alkenyl" is used to mean a linear or branched hydrocarbon group consisting of 2 to 3 carbon atoms containing at least one carbon-carbon double bond, wherein the carbon-carbon double bond can be located at any position within the group. The $C_{2-3}$ alkenyl includes $C_3$ and $C_2$ alkenyl; the $C_{2-3}$ alkenyl can be monovalent, divalent or multivalent. Examples of $C_{2-3}$ alkenyl include, but are not limited to, vinyl, propenyl, *etc.*

Unless otherwise specified, "$C_{2-6}$ alkynyl" is used to mean a linear or branched hydrocarbon group consisting of 2 to 6 carbon atoms containing at least one carbon-carbon triple bond, wherein the carbon-carbon triple bond can be located at any position within the group. The $C_{2-6}$ alkynyl includes $C_{2-4}$, $C_{2-3}$, $C_4$, $C_3$, $C_2$ alkynyl, *etc.* It can be monovalent, divalent or multivalent. Examples of $C_{2-6}$ alkynyl include, but are not limited to, ethynyl, propynyl, butynyl, pentynyl, *etc.*

Unless otherwise specified, "$C_{2-3}$ alkynyl" is used to mean a linear or branched hydrocarbon group consisting of 2 to 3 carbon atoms containing at least one carbon-carbon triple bond, wherein the carbon-carbon triple bond can be located at any position within the group. It can be monovalent, divalent or multivalent. The $C_{2-3}$ alkynyl includes $C_3$ and $C_2$ alkynyl. Examples of $C_{2-3}$ alkynyl include, but are not limited to, ethynyl, propynyl, *etc.*

Unless otherwise specified, "$C_{3-6}$ cycloalkyl" refers to a saturated monocyclic hydrocarbon group consisting of 3 to 6 carbon atoms, and the $C_{3-6}$ cycloalkyl includes $C_{3-4}$, $C_{3-5}$, $C_{4-5}$, $C_{4-6}$, $C_{5-6}$ cycloalkyl, *etc;* it can be monovalent, divalent or multivalent. Examples of $C_{3-6}$ cycloalkyl include, but are not limited to, cyclopropyl, cyclobutyl, cyclopentyl, cyclohexyl, *etc.*

Unless otherwise specified, "$C_{3-4}$ cycloalkyl" refers to a saturated cyclic hydrocarbon group consisting of 3 to 4 carbon atoms, which is a monocyclic system, and the $C_{3-4}$ cycloalkyl includes $C_3$, $C_4$ cycloalkyl, *etc.;* it can be monovalent, divalent or multivalent. Examples of $C_{3-4}$ cycloalkyl include, but are not limited to, cyclopropyl, cyclobutyl, *etc.*

Unless otherwise specified, "3- to 6-membered heterocyclyl" by itself or in combination with other terms refers to a saturated or partially unsaturated monocyclic group consisting of 3 to 6 ring atoms, wherein 1, 2, 3 or 4 ring atoms are heteroatoms independently selected from O, S and N, and the rest are carbon atoms, wherein nitrogen atoms are optionally quaternized, and nitrogen and sulfur heteroatoms can be optionally oxidized (*i.e.,* NO and $S(O)_p$, p is 1 or 2). In addition, with regard to the "3- to 6-membered heterocyclyl", a heteroatom may occupy the connection position of the heterocyclyl with the rest of the molecule. The 3- to 6-membered heterocyclyl includes 4- to 6-membered, 5- to 6-membered, 4-membered, 5-membered, 6-membered heterocyclyl, *etc.* Examples of 3- to 6-membered heterocyclyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, *etc.),* tetrahydrofuranyl (including tetrahydrofuran-2-yl, *etc.),* tetrahydropyranyl, piperidinyl (including 1-piperidinyl, 2-piperidinyl, 3-piperidinyl, *etc.),* piperazinyl (including 1-piperazinyl, 2-piperazinyl, *etc.),* morpholinyl (including 3-morpholinyl, 4-morpholinyl, *etc.),* dioxinyl, dithianyl, isoxazolidinyl, isothiazolidinyl, 1,2-oxazinyl, 1,2-thiazinyl, hexahydropyridazinyl,

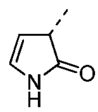, 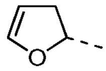, 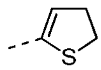,

*etc.*

Unless otherwise specified, "4- to 6-membered heterocycloalkyl" refers to a fully saturated 4- to 6-membered heterocyclyl. The 4- to 6-membered heterocycloalkyl includes 4- to 5-membered, 5- to 6-membered, 4-membered, 5-membered, 6-membered heterocycloalkyl, *etc.* Non-limiting examples of 4-membered heterocycloalkyl include, but are not limited to, azetidinyl, oxetanyl, thietanyl, examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, tetrahydropyrazolyl, and examples of 6-membered heterocycloalkyl include, but are not limited to, piperidinyl, tetrahydropyranyl, thianyl, morpholinyl, piperazinyl, 1,4-oxathianyl, 1,4-dioxanyl, thiomorpholinyl, 1,3-dithianyl, 1,4-dithianyl.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, and any range from n to n+m is also included, for example $C_{1-12}$ includes $C_{1-6}$, $C_{1-6}$, $C_{1-9}$, $C_{3-6}$, $C_{3-9}$, $C_{3-12}$, $C_{6-9}$, $C_{6-12}$, $C_{9-12}$, *etc.;* similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, *etc.*

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuK$\alpha$ radiation as the light source and scanning mode: $\varphi/\omega$ scan, and after collecting the relevant data, the crystal structure is further analyzed by direct method (Shelxs97), so that the absolute configuration can be confirmed.

The solvents used in the present disclosure are commercially available. The compounds of the present disclosure are named according to the conventional naming principles in the art or by ChemDraw® software, and the commercially available compounds use the supplier catalog names.

## BRIEF DESCRIPTION OF THE DRAWINGS

FIG. 1 shows the experimental results of *in vivo* PD model of LPS-induced complement activation in mice.

## DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific embodiments have also been disclosed; for those skilled in the art, it is obvious to make various modifications and improvements to the specific embodiments of the present disclosure without departing from the spirit and scope of the present disclosure.

**Reference Example 1: Synthesis of Intermediate N-c**

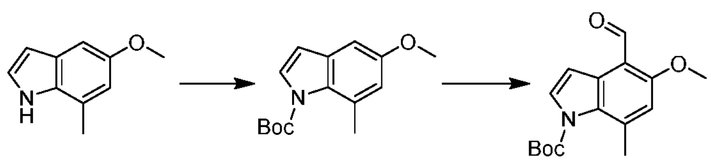

**N-a** **N-b** **N-c**

Step 1

To a solution of compound **N-a** (0.5 g, 3.10 mmol) in tetrahydrofuran (5 mL) were added di-tert-butyl dicarbonate (812.33 mg, 3.72 mmol) and *N,N*-diisopropylethylamine (37.89 mg, 310.17 μmol) at 15°C. The reaction mixture was stirred at 15°C for 1 hour. The reaction mixture was added to water (15 mL) and stirred for another 5 minutes. The phases were separated, and the aqueous phase was extracted three times with ethyl acetate (20 mL). The combined organic layers were washed twice with saturated brine (20 mL), dried over anhydrous sodium sulfate, filtered, and the filtrate was concentrated under reduced pressure. The residue was purified by silica gel column chromatography (ethyl acetate: petroleum ether = 0:1 to 1:10) to obtain compound N-b. $^1$H NMR (400 MHz, $CDCl_3$) δ ppm 7.54-7.48 (m, 1H), 6.90-6.84 (m, 1H), 6.77-6.70 (m, 1H), 6.50-6.43 (m, 1H), 3.90-3.77 (m, 3H), 2.70-2.55 (m, 3H), 1.66-1.61 (m, 9H); LC-MS: *m/z* = 206.1 $[M-56+H]^+$.

Step 2

To a solution of N-methylformanilide (459.93 mg, 3.40 mmol) in dichloromethane (2.7 mL) was slowly added dropwise oxalyl chloride (431.90 mg, 3.40 mmol) under nitrogen atmosphere at 15°C. After the addition was completed, the reaction mixture was stirred at 15°C for 12 hours, and then added dropwise to a solution of compound **N-b** (0.684 g, 2.62 mmol) in dichloromethane (2.8 mL) at -14°C. After the addition was completed, the reaction mixture was stirred at -15°C for another 1.5 hours. The reaction mixture was poured into ice water (20 mL) and stirred for another 5 minutes. The aqueous phase was extracted three times with ethyl acetate (20 mL), and the combined organic phases were washed three times with saturated brine (20 mL), dried over anhydrous sodium sulfate, and filtered. The filtrate was concentrated under reduced pressure, and the residue was purified by silica gel column chromatography (ethyl acetate: petroleum ether = 0:1 to 1:10) to obtain compound **N-c.** $^1$H NMR (400 MHz, DMSO-$d_6$) δ ppm 10.61-10.41 (m, 1H), 7.91-7.71 (m, 1H), 7.38-7.21 (m, 1H), 7.07-6.97 (m, 1H), 3.97-3.93 (m, 3H), 2.63-2.59 (m, 3H), 1.60-1.58 (m, 9H); LC-MS: *m/z* =290.1 $[M+H]^+$.

**Reference Example 2: Synthesis of Intermediate M-c**

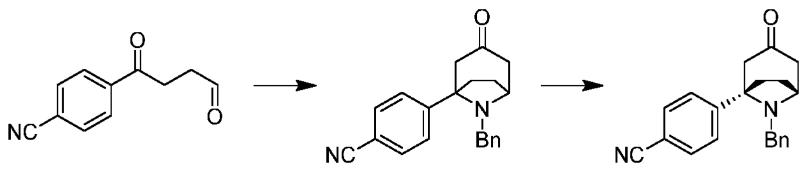

**M-a** **M-b** **M-c**

Step 1

Benzylamine (194.62 g, 1.82 mol) was slowly added to water (850 mL) in which acetic acid (103.88 mL, 1.82 mol) was dissolved. The reaction system was cooled to 0 to 10°C, and then 1,3-acetonedicarboxylic acid (265.36 g, 1.82 mol) was slowly added in batches, and the reaction mixture was reacted at 0°C for 0.5 hours. To the reaction system was slowly added a solution of compound **M-a** (170 g, 908.15 mmol) dissolved in dioxane (850 mL), and the reaction mixture was slowly returned to room temperature, and then reacted at 45°C for 12 hours. The reaction mixture was cooled to room temperature, then ethyl acetate was added for extraction (500 mL * 2). The organic phases were combined, washed sequentially with saturated sodium bicarbonate (500 mL) and saturated sodium chloride aqueous solution (500 mL), dried over anhydrous sodium sulfate, and the filtrate was concentrated under reduced pressure to obtain a crude product. The crude product was stirred in a mixed solvent of n-heptane and methyl *tert*-butyl ether (1:1, 500 mL) for 0.5 hours and filtered, and the solid was collected to obtain compound **M-b.** LC-MS: *m/z* = 317.1 $[M+H]^+$.

Step 2

Compound **M-b** (220 g, 696.20 mmol) was dissolved in acetonitrile (1700 mL), then heated to 70°C, and stirred for 2 hours. Dibenzoyl-L-tartaric acid (200 g, 558.66 mmol) was slowly added with stirring, and the reaction mixture was stirred at 70°C for another 2 hours, then slowly returned to 25°C, and stirred for 12 hours. The reaction mixture was filtered, and the solid was collected. The filter cake was washed with acetonitrile (400 mL * 2). The solid obtained by filtration was added to 1000 mL of water. The pH was adjusted to 8 by adding 1 M NaOH aqueous solution with stirring, and then the mixture was extracted with ethyl acetate (2000 mL * 2). The organic phases were combined, washed with saturated brine (2000 mL * 2),

dried over anhydrous sodium sulfate, and filtered. The filtrate was concentrated under reduced pressure to obtain compound **M-c** (retention time: 2.175 min, ee = 99.1%). LC-MS: *m/z* = 317.1 [M+H]+. SFC analysis method: chromatographic column: ChiralpakAD 50 × 4.6 mm I.D., 3 μm, mobile phase: A: $CO_2$, B: methanol (0.05% diethylamine), gradient B%: 5% to 40%.

**Example 1**

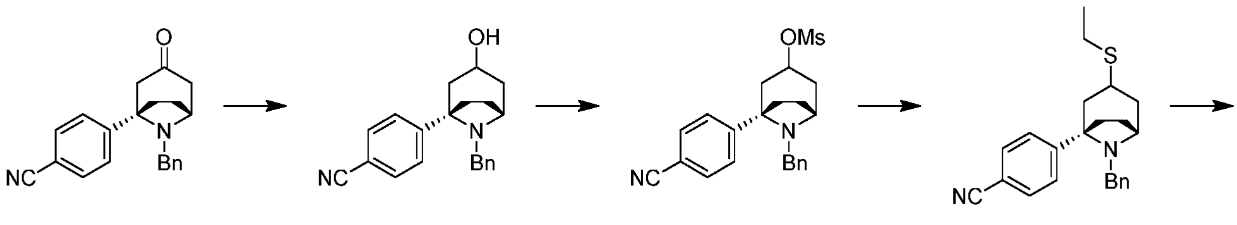

**M-c** **1-1** **1-2** **1-3**

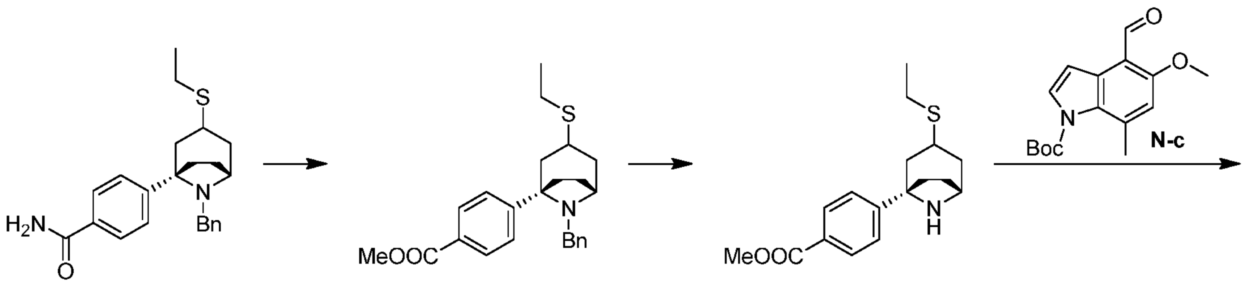


**1-4** **1-5** **1-6**

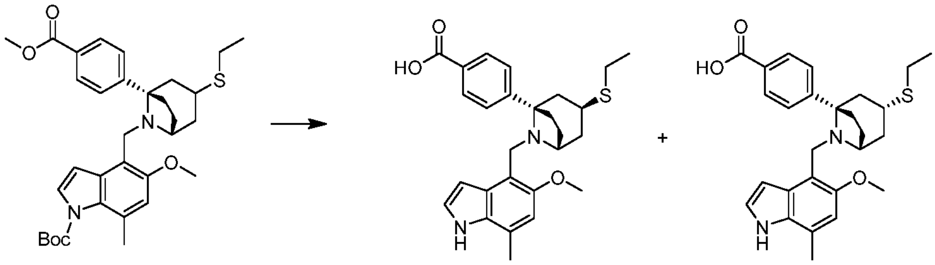

**1-7** **1A or 1B** **1A or 1B**

Step 1

To a solution of compound **M-c** (30 g, 94.82 mmol) in methanol (150 mL) and acetonitrile (150 mL) was added sodium borohydride (3.95 g, 104.30 mmol) in batches at 0°C. The reaction mixture was slowly warmed to 20°C and stirred at 20°C for 2 hours. 1 M dilute hydrochloric acid solution was slowly added dropwise to the reaction mixture to quench the reaction until no bubbles were generated. The pH was then adjusted to 7 to 8 with saturated sodium bicarbonate aqueous solution, and extracted with ethyl acetate (500 mL * 2). The combined organic phases were washed with saturated brine (600 mL * 2), dried over anhydrous sodium sulfate, and filtered. The filtrate was concentrated under reduced pressure to obtain a crude product. To a solution of crude product (27 g, 84.80 mmol) in methyl tert-butyl ether (135 mL) was slowly added hydrochloric acid/ethyl acetate (4 M, 31.80 mL) at 25°C. The mixture was stirred at 25°C for another 2 hours. A solid was precipitated, and the mixture was filtered to collect the solid. Water (200 mL) and methyl *tert*-butyl ether (300 mL) were then added to the resulting solid, and the pH was adjusted to 7 to 8 with sodium hydroxide aqueous solution (4 M). The mixture was extracted with methyl *tert*-butyl ether (300 mL), and the organic phase was washed with saturated brine (200 mL), dried over anhydrous sodium sulfate, and filtered. The filtrate was concentrated under reduced pressure to obtain compound **1-1.** LC-MS: *m/z* = 319.3 [M+H]+.

Step 2

Compound **1-1** (10 g, 31.41 mmol) and triethylamine (4.77 g, 47.11 mmol) were dissolved in the solvent dichloromethane (100 mL) and cooled to -20°C. Methanesulfonyl chloride (5.32 g, 46.48 mmol) was slowly added dropwise, and the reaction mixture was stirred at -20°C for 1 hour. The reaction was quenched by slowly adding 200 mL of water at -20°C. Then dichloromethane (250 mL * 2) was added for extraction. The organic phases were combined and washed with saturated brine (200 mL), dried over anhydrous sodium sulfate, and filtered. The filtrate was concentrated under reduced pressure. The residue was purified by silica gel column chromatography (ethyl acetate: petroleum ether = 1:10 to 1:3) to obtain compound **1-2.** LC-MS: *m*/*z* = 397.1 $[M+H]^+$.

Step 3

Ethanethiol (8.94 g, 143.82 mmol) was dissolved in a solution of tetrahydrofuran (60 mL), and sodium hydride (6.14 g, 127.84 mmol, purity of 50%) was added under nitrogen atmosphere. The reaction mixture was stirred at 20°C for 0.5 hours. To the reaction mixture was then slowly added a solution of compound **1-2** (6.4 g, 15.98 mmol) dissolved in *N*,*N*-dimethylformamide (60 mL). The reaction mixture was stirred at 70°C for 2 hours. The reaction was quenched by adding 100 mL of saturated ammonium chloride aqueous solution to the reaction mixture at 0°C and then diluted by adding 100 mL of water. Ethyl acetate (500 mL * 2) was added for extraction. The organic phases were combined and washed with saturated brine (500 mL), dried over anhydrous sodium sulfate, and filtered. The filtrate was concentrated under reduced pressure. The residue was purified by silica gel column chromatography (ethyl acetate: petroleum ether = 1:20 to 1:8) to obtain compound **1-3.** LC-MS: *m*/*z* = 363.1 $[M+H]^+$.

Step 4

Compound **1-3** (1.5 g, 4.14 mmol) was dissolved in *tert*-butanol (25 mL), then potassium tert-butoxide (1.39 g, 12.41 mmol) was added thereto, and the reaction mixture was stirred at 70°C for 0.5 hours. The reaction mixture was cooled to 20°C, then the reaction mixture was diluted by adding 100 mL of water, and extracted by adding ethyl acetate (200 mL * 2). The organic phases were combined, washed with saturated brine (100 mL * 2), dried over anhydrous sodium sulfate, and filtered. The filtrate was concentrated under reduced pressure to obtain compound **1-4.** LC-MS: *m*/*z* = 381.1 $[M+H]^+$.

Step 5

Compound **1-4** (1.5 g, 3.94 mmol) was dissolved in methanol (30 mL), then *N,N*-dimethylformamide dimethyl acetal (1.41 g, 11.83 mmol) was added thereto, and the reaction mixture was stirred at 70°C for 12 hours. The reaction mixture was cooled to 20°C, then the reaction mixture was diluted by adding 100 mL of water, and extracted by adding ethyl acetate (200 mL * 2). The organic phases were combined, washed with saturated brine (100 mL * 2), dried over anhydrous sodium sulfate, and filtered. The filtrate was concentrated under reduced pressure to obtain compound **1-5.** LC-MS: *m*/*z* = 396.3 $[M+H]^+$.

Step 6

Compound **1-5** (1.0 g, 2.53 mmol) was dissolved in methanol (30 mL), then concentrated hydrochloric acid (614.51 mg, 5.06 mmol, purity of 37%) and wet palladium on carbon (3 g, purity of 10%) were sequentially added under nitrogen atmosphere. The reaction system was replaced three times with hydrogen and stirred at 50°C under hydrogen atmosphere for 2 hours. The reaction mixture was cooled to 20°C and filtered. The filtrate was concentrated to obtain compound **1-6.** LC-MS: *m*/*z* = 306.1 $[M+H]^+$.

Step 7

Compound **1-6** (400 mg, 1.31 mmol) and compound **N-c** (341.01 mg, 1.18 mmol) were dissolved in 1,4-dioxane (10 mL), and pyridine (124.31 mg, 1.57 mmol) and boron trifluoride diethyl etherate (223.05 mg, 1.57 mmol) were sequentially added thereto. Then polymethylsiloxane (787.43 mg, 13.10 mmol) was added thereto, and the reaction mixture was stirred at 100°C for 12 hours under nitrogen atmosphere. The reaction mixture was cooled to 20°C and diluted by adding 100 mL of water. Ethyl acetate (200 mL * 2) was added for extraction. The organic phases were combined, washed with saturated brine (200 mL * 2), dried over anhydrous sodium sulfate, and filtered. The filtrate was concentrated under reduced pressure. The residue was purified by silica gel column chromatography (ethyl acetate: petroleum ether = 1:20 to 1:5) to obtain compound **1-7.** LC-MS: *m*/*z* = 579.3 $[M+H]^+$.

Step 8

To a solution of compound **1-7** (450 mg, 777.52 μmol) in tetrahydrofuran (5 mL) and methanol (5 mL) was added lithium hydroxide aqueous solution (4 M, 1.94 mL), and the reaction mixture was stirred at 50°C for 2 hours. The pH of the obtained residue after concentration of the reaction mixture was adjusted to 7 with acetic acid, then concentrated under reduced pressure, and the obtained residue was purified by high-performance liquid chromatography (chromatographic column: Phenomenex C18 150 × 40 mm × 15 μm; mobile phase: A: water (0.225% formic acid), B: acetonitrile, method: B: 16% to 46%) to obtain the formate salt of compound **1A** and the formate salt of compound **1B.**

Characterization of the formate salt of compound **1A:** (retention time: 1.692 min, HPLC analysis method: chromatographic column: Kinetex EVO C18 50 × 4.6 mm, 5 μm, mobile phase: A water (0.0375% trifluoroacetic acid), B: acetonitrile (0.01875% trifluoroacetic acid), gradient B%: 10% to 80%), $^1$H NMR (400 MHz, DMSO-$d_6$) δ ppm 10.83 (br s, 1H), 8.18 (s, 1H), 7.98 (d, J = 8.4 Hz, 2H), 7.74 (d, J = 8.4 Hz, 2H), 7.27 (t, J = 2.8 Hz, 1H), 6.67 (s, 1H), 6.47 (d, J = 2.6 Hz, 1H), 3.78 (br d, J = 12.2 Hz, 1H), 3.73 (s, 3H), 3.22 - 3.11 (m, 3H), 2.59 (q, J = 7.4 Hz, 2H), 2.43 (s, 3H), 2.19 - 1.94 (m, 5H), 1.85 (br s, 1H), 1.64 (br d, J = 7.3 Hz, 1H), 1.55 - 1.47 (m, 1H), 1.20 (t, J = 7.4 Hz, 3H); LC-MS: m/z = 465.2 $[M+H]^+$.

Characterization of the formate salt of compound **1B:** (retention time: 1.779 min, HPLC analysis method: chromatographic column: Kinetex EVO C18 50 × 4.6 mm, 5 μm, mobile phase: A water (0.0375% trifluoroacetic acid), B: acetonitrile (0.01875% trifluoroacetic acid), gradient B%: 10% to 80%), $^1$H NMR (400 MHz, DMSO-$d_6$) δ ppm 10.87 (br s, 1H), 8.19 (s, 1H), 7.99 (d, J = 8.4 Hz, 2H), 7.79 (d, J = 8.4 Hz, 2H), 7.28 (t, J = 2.8 Hz, 1H), 6.68 (s, 1H), 6.51 (d, J = 2.6 Hz, 1H), 3.79 (br d, J = 12.2 Hz, 1H), 3.76 (s, 3H), 3.21 - 3.19 (m, 3H), 2.61 (q, J = 7.4 Hz, 2H), 2.47 (s, 3H), 2.19 - 1.95 (m, 5H), 1.88 (br s, 1H), 1.66 (br d, J = 7.3 Hz, 1H), 1.56 - 1.43 (m, 1H), 1.22 (t, J = 7.4 Hz, 3H); LC-MS: m/z = 465.2 $[M+H]^+$.

Compound **1A** has a shorter retention time in the chiral chromatography column relative to **1B,** and compound **1B** has a longer retention time in the chiral chromatography column relative to **1A.**

**Example 2**

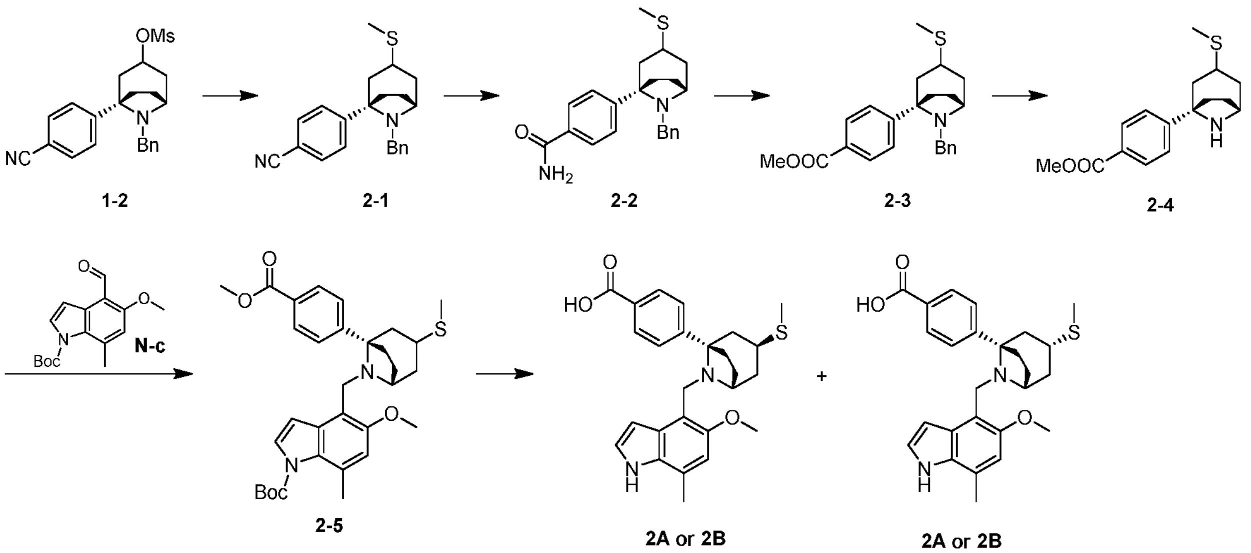

1-2, 2-1, 2-2, 2-3, 2-4

N-c, 2-5, 2A or 2B + 2A or 2B

Step 1

To a solution of compound **1-2** (2 g, 5.04 mmol) in *N,N*-dimethylformamide (30 mL) was added sodium thiomethoxide (1.41 g, 20.18 mmol) at room temperature, and the reaction mixture was heated to 50°C and reacted for 16 hours. The reaction mixture was cooled to room temperature, added with 100 mL of water, then extracted with ethyl acetate (50 mL * 3), washed with saturated brine (60 mL * 2), dried over anhydrous sodium sulfate, filtered, and concentrated to obtain a residue. The residue was purified by silica gel column chromatography (ethyl acetate: petroleum ether = 1:10) to obtain compound **2-1.** LC-MS: m/z = 349.3 $[M+H]^+$.

Step 2

To a solution of compound **2-1** (1.44 g, 4.13 mmol) in *tert*-butanol (20 mL) was added potassium *tert*-butoxide

(1.39 g, 12.40 mmol) at room temperature, and the reaction mixture was heated to 50°C and reacted for 4 hours. The reaction mixture was cooled to room temperature, diluted by adding 20 mL of water, and then extracted with ethyl acetate (50 mL * 3). The organic phases were combined and washed with saturated brine (50 mL * 2), dried over anhydrous sodium sulfate, filtered, and concentrated to obtain compound **2-2.** LC-MS: m/z = 367.3 $[M+H]^+$.

Step 3

To a solution of compound **2-2** (1 g, 2.73 mmol) in methanol (20 mL) was added *N,N*-dimethylformamide dimethyl acetal (975.35 mg, 8.19 mmol), and the reaction mixture was heated to 50°C and reacted for 16 hours. The reaction mixture was cooled to room temperature, concentrated under reduced pressure, and the residue was purified by silica gel column chromatography (ethyl acetate: petroleum ether = 1:10) to obtain compound **2-3.** LC-MS: m/z = 382.3 $[M+H]^+$.

Step 4

To a solution of compound **2-3** (0.8 g, 2.10 mmol) in methanol (10 mL) under nitrogen atmosphere were added wet palladium on carbon (1 g, purity of 10%) and concentrated hydrochloric acid (254.84 mg, 2.10 mmol, purity of 37%). The reaction system was replaced three times with hydrogen and reacted at 60°C under hydrogen atmosphere for 6 hours. The reaction mixture was cooled to room temperature, filtered, and the filtrate was concentrated under reduced pressure to obtain compound **2-4.** LC-MS: m/z = 292.4 $[M+H]^+$.

Step 5

To a solution of compound **2-4** (300 mg, 1.03 mmol) in 1,4-dioxane (3 mL) were added **N-c** (357.43 mg, 1.24 mmol), pyridine (97.72 mg, 1.24 mmol), boron trifluoride diethyl etherate (175.34 mg, 1.24 mmol) and polymethylsiloxane (619.03 mg, 10.29 mmol) at room temperature, and the reaction mixture was heated to 100°C and reacted for 16 hours. The reaction mixture was cooled to room temperature and filtered. The filter cake was washed with ethyl acetate, and the filtrate was washed sequentially with citric acid (1 mL * 2), saturated sodium bicarbonate (1 mL * 2) and saturated brine (1 mL * 2), dried over anhydrous sodium sulfate, filtered, and concentrated to obtain a residue. The residue was purified by silica gel column chromatography (ethyl acetate: petroleum ether = 1:10) to obtain compound **2-5.** LC-MS: m/z = 565.4 $[M+H]^+$.

Step 6

To a solution of compound **2-5** (0.37 g, 655.18 μmol) in methanol (3 mL) and tetrahydrofuran (3 mL) was added lithium hydroxide aqueous solution (4 M, 3 mL), and the reaction mixture was stirred at 50°C for 3 hours. After the reaction was completed, the reaction mixture was cooled to room temperature, and the pH was adjusted to about 7 by adding acetic acid. The mixture was concentrated to obtain a residue. The residue was purified by high-performance liquid chromatography (chromatographic column: Phenomenex luna C18 250 × 80 mm, 10 μm; mobile phase: A: water (0.225% formic acid); B: acetonitrile; method B: 30% to 60%) to obtain the formate salt of compound **2A** and the formate salt of compound **2B.**

Characterization of the formate salt of compound **2A:** (retention time: 1.612 min, HPLC analysis method: chromatographic column: Kinetex EVO C18 50 × 4.6 mm, 5 μm, mobile phase: A water (0.0375% trifluoroacetic acid), B: acetonitrile (0.01875% trifluoroacetic acid), gradient B%: 10% to 80%), $^1H$ NMR (400 MHz, DMSO-$d_6$) δ ppm 10.89 - 10.71 (m, 1H), 8.03 - 7.88 (m, 2H), 7.80 - 7.67 (m, 2H), 7.29 - 7.20 (m, 1H), 6.69 - 6.58 (m, 1H), 6.50 - 6.39 (m, 1H), 3.82 - 3.74 (m, 1H), 3.73 - 3.67 (m, 3H), 3.18 - 3.11 (m, 2H), 3.10 - 2.97 (m, 2H), 2.43 - 2.37 (m, 3H), 2.13 - 2.08 (m, 2H), 2.08 - 2.06 (m, 3H), 2.05 - 1.97 (m, 3H), 1.92 - 1.76 (m, 1H), 1.71 - 1.52 (m, 1H), 1.52 - 1.37 (m, 1H); LC-MS: m/z = 451.3 $[M+H]^+$.

Characterization of the formate salt of compound **2B:** (retention time: 1.677 min, HPLC analysis method: chromatographic column: Kinetex EVO C18 50 × 4.6 mm, 5 μm, mobile phase: A water (0.0375% trifluoroacetic acid), B: acetonitrile (0.01875% trifluoroacetic acid), gradient B%: 10% to 80%), $^1H$ NMR (400 MHz, DMSO-$d_6$) δ ppm 10.90 - 10.68 (m, 1H), 8.15 - 8.07 (m, 1H), 7.99 - 7.87 (m, 2H), 7.79 - 7.67 (m, 2H), 7.30 - 7.17 (m, 1H), 6.72 - 6.59 (m, 1H), 6.52 - 6.40 (m, 1H), 3.78 - 3.70 (m, 1H), 3.70 - 3.66 (m, 3H), 3.22 - 3.13 (m, 3H), 3.14 - 3.03 (m, 2H), 2.41 - 2.36 (m, 3H), 2.35 - 2.23 (m, 1H), 2.18 - 2.08 (m, 2H), 2.07 (s, 3H), 2.02 - 1.89 (m, 2H), 1.50 - 1.36 (m, 1H); LC-MS: m/z = 451.3 $[M+H]^+$.

Compound **2A** has a shorter retention time in the chiral chromatography column relative to **2B,** and compound **2B** has a longer retention time in the chiral chromatography column relative to **2A.**

**Example 3**

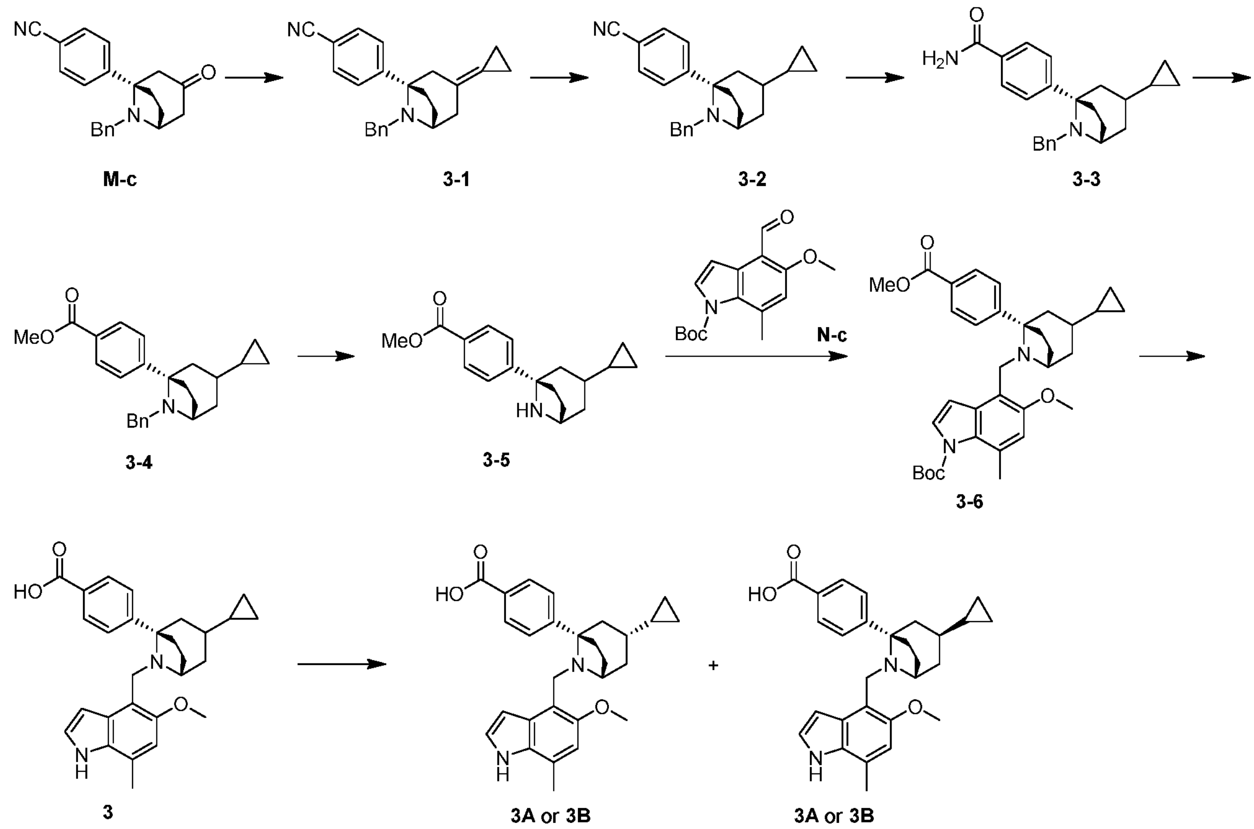
M-c 3-1 3-2 3-3
3-4 3-5 N-c 3-6
3 3A or 3B + 3A or 3B

Step 1

To a solution of compound **M-c** (2 g, 6.32 mmol) and (3-bromopropyl)triphenylphosphonium bromide (3.81 g, 8.22 mmol) in tetrahydrofuran (30 mL) was added potassium *tert*-butoxide (1.84 g, 16.44 mmol) at room temperature, and the reaction mixture was heated to 50°C and reacted for 16 hours under nitrogen atmosphere. After the reaction was completed, the reaction mixture was cooled to room temperature, added with 100 mL of water, then extracted with ethyl acetate (100 mL), washed with saturated brine (100 mL), dried over anhydrous sodium sulfate, filtered, and concentrated to obtain a residue. The residue was purified by silica gel column chromatography (ethyl acetate: petroleum ether = 0:1 to 1:15) to obtain compound **3-1.** LC-MS: m/z = 341.2 $[M+H]^+$.

Step 2

To a solution of compound **3-1** (1 g, 2.94 mmol) in *N,N*-dimethylformamide (10 mL) was added benzenesulfonyl hydrazide (1.52 g, 8.81 mmol) at room temperature, and the reaction mixture was heated to 100°C and reacted for 3 hours. The reaction mixture was cooled to room temperature, diluted by adding 50 mL of water, and then extracted with ethyl acetate (50 mL). The organic phases were combined, washed with saturated brine (50 mL), dried over anhydrous sodium sulfate, filtered, and concentrated to obtain a residue. The residue was purified by silica gel column chromatography (ethyl acetate: petroleum ether = 0:1 to 1:10) to obtain compound **3-2.** LC-MS: m/z = 343.4 $[M+H]^+$.

Step 3

To a solution of compound **3-2** (0.62 g, 1.81 mmol) in dimethyl sulfoxide (5 mL) were added potassium carbonate (275.22 mg, 1.99 mmol) and hydrogen peroxide (307.89 mg, 2.72 mmol, 30% aqueous solution) at room temperature, and the reaction mixture was heated to 40°C and reacted for 5 hours. The reaction mixture was cooled to room temperature, added to 20 mL of water to precipitate a solid, and filtered to collect the solid. The solid was dried to obtain compound **3-3.** LC-MS: m/z = 361.4 $[M+H]^+$.

Step 4

To a solution of compound **3-3** (0.62 g, 1.72 mmol) in methanol (25 mL) was added *N,N*-dimethylformamide

dimethyl acetal (614.83 mg, 5.16 mmol), and the reaction mixture was heated to 60°C and reacted for 16 hours. The reaction mixture was cooled to room temperature, diluted by adding 30 mL of water, and then extracted with ethyl acetate (30 mL). The organic phases were combined and washed with saturated brine (30 mL), dried over anhydrous sodium sulfate, filtered, and concentrated to obtain compound **3-4.** LC-MS: m/z = 376.4 $[M+H]^+$.

Step 5

To a solution of compound **3-4** (0.6 g, 1.60 mmol) in methanol (15 mL) were added wet palladium on carbon (0.6 g, purity of 10%) and concentrated hydrochloric acid (266.31 μL, purity of 37%) under nitrogen atmosphere. The reaction system was replaced three times with hydrogen and reacted at 50°C for 3 hours under hydrogen atmosphere. The reaction mixture was cooled to room temperature and filtered, and the filtrate was concentrated. The residue was diluted by adding ethyl acetate (15 mL), adjusted to pH = 9 with saturated sodium bicarbonate, extracted with ethyl acetate (15 mL), washed with saturated brine (15 mL), dried over anhydrous sodium sulfate, filtered, and concentrated to obtain compound **3-5.** LC-MS: m/z = 286.4 $[M+H]^+$.

Step 6

To a solution of compound **3-5** (0.29 g, 1.02 mmol) in 1,4-dioxane (5 mL) were added compound **N-c** (382.21 mg, 1.32 mmol), pyridine (96.46 mg, 1.22 mmol), boron trifluoride diethyl etherate (173.07 mg, 1.22 mmol) and polymethylsiloxane (609.71 mg, 10.16 mmol) at room temperature, and the reaction mixture was heated to 90°C and reacted for 16 hours. The reaction mixture was cooled to room temperature, diluted by adding 20 mL of water, and then extracted with ethyl acetate (20 mL). The organic phases were combined and washed with saturated brine (20 mL), dried over anhydrous sodium sulfate, filtered, and concentrated to obtain a residue. The residue was purified by silica gel column chromatography (ethyl acetate: petroleum ether = 1:20 to 1:5) to obtain compound **3-6.** LC-MS: *m/z* = 559.3 $[M+H]^+$.

Step 7

To a solution of compound **3-6** (0.51 g, 912.82 μmol) in methanol (5 mL) and tetrahydrofuran (5 mL) was added lithium hydroxide aqueous solution (4 M, 5 mL), and the reaction mixture was stirred at 50°C for 3 hours. After the reaction was completed, the reaction mixture was cooled to room temperature, and the pH was adjusted to about 7 by adding acetic acid. The mixture was concentrated to obtain a residue. The residue was purified by high-performance liquid chromatography (chromatographic column: Phenomenex luna C18 250 × 50 mm, 10 μm; mobile phase: A: water (0.225% formic acid); B: acetonitrile; method B: 25% to 40%) to obtain compound **3.** LC-MS: m/z = 445.3 $[M+H]^+$.

Step 8

Compound **3** was separated by chiral column (chromatographic column: Daicel ChiralPak IC 250 × 30 mm, 10 μm, mobile phase: A: carbon dioxide, B: isopropanol (0.1% ammonia water), method: B: 40% to 40%) to obtain compound **3A** and compound **3B.**

Characterization of compound **3A:** (retention time: 4.007 min, ee = 100%, SFC analysis method: chromatographic column: Chiralpak IC-3 50 × 4.6 mm I.D., 3 μm, mobile phase: A: carbon dioxide, B: 40% methanol + 60% acetonitrile (0.05% diethylamine), gradient B%: 0% to 40%), $^1H$ NMR (400 MHz, DMSO-$d_6$) δ ppm 10.81 (s, 1H), 7.98 - 7.96 (d, J = 8.0 Hz, 2H), 7.76 - 7.74 (d, J = 8.0 Hz, 2H), 7.36 - 7.16 (m, 1H), 6.71 - 6.63 (m, 1H), 6.55 - 6.50 (m, 1H), 3.80 - 3.74 (m, 4H), 3.49 - 3.41 (m, 2H), 3.23 - 3.14 (m, 3H), 2.42 (s, 3H), 2.19 - 1.94 (m, 2H), 1.68 - 1.58 (m, 1H), 1.55 - 1.46 (m, 1H), 1.32 - 1.18 (m, 2H), 0.68 - 0.50 (m, 1H), 0.39 - 0.26 (m, 2H), 0.15 - 0.05 (m, 2H); LC-MS: m/z = 445.3 $[M+H]^+$.

Characterization of compound **3B:** (retention time: 4.505 min, ee = 100%, SFC analysis method: chromatographic column: Chiralpak IC-3 50 × 4.6 mm I.D., 3 μm, mobile phase: A: carbon dioxide, B: 40% methanol + 60% acetonitrile (0.05% diethylamine), gradient B%: 0% to 40%), $^1H$ NMR (400 MHz, DMSO-$d_6$) δ ppm 10.82 (s, 1H), 8.01 - 7.88 (d, J = 8.0 Hz, 2H), 7.79 - 7.68 (d, J = 8.0 Hz, 2H), 7.31 - 7.21 (m, 1H), 6.70 - 6.62 (m, 1H), 6.59 - 6.52 (m, 1H), 3.81 - 3.73(m, 4H), 3.49 - 3.41 (m, 2H), 3.23 - 3.14 (m, 3H), 2.43 (s, 3H), 2.33 - 2.06 (m, 2H), 1.89 - 1.76 (m, 2H), 1.34 - 1.17 (m, 2H), 0.99 - 0.90 (m, 1H), 0.48 - 0.37 (m, 2H), 0.04 -0.06 (m, 2H); LC-MS: m/z = 445.3 $[M+H]^+$.

Compound **3A** has a shorter retention time in the chiral chromatography column relative to **3B,** and compound **3B** has a longer retention time in the chiral chromatography column relative to **3A.**

**Biological Test Section**

**Experimental Example 1: Complement Human Factor B Protein Binding Test (Surface Plasmon Resonance (SPR) Method)**

Experimental purpose: To detect the kinetic constants and affinity of the binding between the test compounds and human complement Factor B using the SPR method.

Experimental scheme:

1-Ethyl-3-(3-dimethylaminopropyl) carbodiimide hydrochloride (EDC) and *N*-hydroxysulfosuccinimide sodium salt (NHS) were mixed in a 1:1 ratio, and complement Factor B was diluted to 20 μg/mL in acetic acid solution with a pH of 5.5. Using a biomacromolecule interaction analyzer Biacore 8K, the programmed settings were set to add the EDC/NHS, the FB protein solution and the 1 M ethanolamine-hydrochloric acid solution to the detection plate. The detection plate was placed in the machine detection chamber, and the EDC/NHS mixture, the complement Factor B protein solution and the 1 M ethanolamine-hydrochloric acid solution were sequentially injected onto the surface of the CM5 chip according to the procedure for chip activation, ligand coupling and deactivation. The flow cell 1 of each channel was used as a reference channel for surface activation and deactivation only, without injection of complement Factor B protein solution. All sample stock solutions were diluted with running buffer (PBST), and all samples were serially diluted 2 times in duplicate for 6 points. The prepared sample was transferred to the detection plate according to the program settings and the detection plate was placed in the sample chamber of Biacore 8K. The detection program was run, and the samples were injected into each channel of the chip sequentially according to the program, with a binding time of 120 s, a dissociation time of 600 s and a flow rate of 30 μL/min. The data were analyzed using Biacore Insight Evaluation Software: The data were fitted using a 1:1 Binding Kinetics Model to obtain the binding rate constant (kon), dissociation rate constant (koff) and dissociation equilibrium constant (KD) for binding of each test sample to complement Factor B protein.

Experimental results:

The binding activity of the test compounds to human complement Factor B protein is shown in Table 1. Where A represents: $IC_{50}$ value < 100 nM; B represents: 100 nM ≤ $IC_{50}$ value ≤ 1000 nM; C represents: $IC_{50}$ value > 1000 nM.

**Table 1: *In vitro* human complement Factor B protein binding activity screening test results of the compounds of the present disclosure**

| Compound | **Human complement Factor B protein binding KD (nM)** |
|---|---|
| Formate salt of compound **1A** | A |
| Formate salt of compound **1B** | A |
| Formate salt of compound **2A** | A |
| Formate salt of compound **2B** | A |
| Compound **3A** | A |
| Compound **3B** | A |

Conclusion: The compounds of the present disclosure have significant binding activity to human complement Factor B protein.

**Experimental Example 2: *In Vivo* PD Model of LPS-induced Complement Activation in Mice**

Experimental objective: To investigate the inhibitory effect of the compounds of the present disclosure on complement activation in mice induced by LPS stimulation.

Experimental animals: female C57BL/6J mice, 7 to 9 weeks old, weighing 17 to 23 g; supplier: Shanghai Sipple-Bikai Laboratory Animal Co., Ltd.

Experimental procedure:

4 hours before administration, 100 μg of lipopolysaccharide (LPS) in *Escherichia coli* (Sigma, L2880) dissolved in 100 μL of sterile PBS (phosphate buffered saline, pH 7.2 to 7.4) was intraperitoneally injected to induce complement activation in mice. Normal mouse group (negative control): Animals were intraperitoneally injected with 100 μL of sterile

PBS, and the vehicle alone was administered by gavage (IO). Model group (positive control): Animals were intraperitoneally injected with LPS and the vehicle was administered by PO (20% PEG400/10% solutol/70% water). Drug group: Sample collection was performed 4 hours after administration of the compound (vehicle: 20% PEG400/10% solutol/70% water, administration volume: 5 mL/kg, dose: 10 mg/kg).

Sample collection: 0.3 mL of blood samples were collected from the orbital venous plexus. All blood samples were added to 1.5 mL commercial EDTA-K2 anticoagulant tubes (supplied by Jiangsu Kangjian Medical Apparatus Co., Ltd.). After collection, within half an hour, the blood sample was centrifuged at 4°C, 3000 g for 10 min to aspirate the supernatant plasma, which was put in dry ice immediately, and stored in a refrigerator at -80°C for Western blot analysis of downstream C3d protein levels after complement activation.

Sample analysis: Mouse plasma (5 μL) + Lysis buffer (27.5 μL) + Loading buffer (12.5 μL) + Reducing buffer (5 μL) were mixed well and incubated at 100°C for 15 min with a loading volume of 5 μL/well, *i.e.*, a loading volume of plasma of 0.5 μL per well.

Experimental results:

After complement activation was induced by intraperitoneal injection, the level of C3d protein in the serum of mice in the model group increased by 60% to 90% compared with that in the normal group. After oral administration of the compound of the present disclosure, the level of C3d protein in the serum of mice in each drug group decreased by 70% to 90% compared with that in the model group, and the level of C3d protein decreased significantly. The specific experimental results are shown in FIG. 1.

Experimental conclusion: Compared with the normal group and model group, the compounds of the present disclosure can significantly inhibit complement activation stimulated by LPS.

**Experimental Example 3: Plasma Protein Binding Rate Test of Compounds**

Experimental purpose: To evaluate the protein binding rate of the compounds of the present disclosure in the plasma of CD-1 mice, SD rats, beagle dogs, cynomolgus monkeys and humans using equilibrium dialysis.

Experimental scheme:

The test compounds were diluted with dialysis buffer into the plasma of the above five species to prepare samples with a final concentration of 2 μM. The samples were then added to a 96-well equilibrium dialysis device and dialyzed using phosphate buffered saline solution at 37°C for 4 hours. Warfarin was used as a control compound in the experiment. The concentration of the test compounds and warfarin in the plasma and buffer was determined using LC-MS/MS. The free fraction of the compound was calculated by the following formula: PPB_Unbound (%) = 100 * $F_C/T_C$, where $F_C$ is the concentration of the compound at the buffer end of the dialysis plate; $T_C$ is the concentration of the compound at the plasma end of the dialysis plate; $T_0$ is the concentration of the compound in the plasma sample at time zero. The experimental results are shown in Table 2.

Table 2: Results of plasma protein binding rate of the compounds of the present disclosure

| Compound | Plasma protein binding (PPB) unbound (%) | | | | |
|---|---|---|---|---|---|
| | Mouse (M) | Rat (R) | Dog (D) | Cynomolgus monkey (C) | Human (H) |
| Compound **1B** | 12.5 | 11.6 | 16.2 | 17.4 | 13.3 |
| Compound **2B** | 22.2 | 15.1 | 31.7 | 25.6 | 21.7 |
| Compound **3B** | 19.1 | 14.1 | 25.6 | 28.5 | 15.2 |

Conclusion: The compounds of the present disclosure have moderate plasma protein binding rates in the plasma of the various species, which indicates that in the plasma of the various species, the compounds of the present disclosure have moderate free drug concentration ratios, and have good druggability.

**Experimental Example 4: *In Vitro* Liver Microsomal Stability Test of Compounds**

Experimental materials

Liver microsomes: purchased from Corning or Xenotech, stored in a -80°C refrigerator; reduced nicotinamide

adenine dinucleotide phosphate (NADPH), supplier: Chem-Impex International, Cat. No.: 00616; control compounds: testosterone, diclofenac, propafenone.

Experimental steps

(1) Preparation of working solutions

Stock solution: 10 mM DMSO solution; preparation of working concentration: The stock solution was diluted to 100 μM using 100% acetonitrile (organic phase content: 99% acetonitrile, 1% DMSO).

(2) Experimental steps

Two 96-well incubation plates were prepared and named as T60 incubation plate and NCF60 incubation plate.

445 μL of microsomal working solution (liver microsomal protein concentration of 0.56 mg/mL) was added to both the T60 incubation plate and the NCF60 incubation plate. The two incubation plates were pre-incubated in a 37°C water bath for approximately 10 minutes.

After pre-incubation was completed, 5 μL of the test compound or control compound working solution was added to both the T60 incubation plate and the NCF60 incubation plate and mixed well. 50 μL of potassium phosphate buffer per well was added to the NCF60 incubation plate to initiate the reaction. 180 μL of termination solution (acetonitrile solution containing 200 ng/mL tolbutamide and 200 ng/mL labetalol) and 6 μL of NADPH regeneration system working solution were added to a T0 termination plate. 54 μL of sample was then removed from the T60 incubation plate and added to the T0 termination plate to generate T0 samples. 44 μL of NADPH regeneration system working solution per well was added to the T60 incubation plate to initiate the reaction. Only 54 μL of the microsomal working solution, 6 μL of the NADPH regeneration system working solution and 180 μL of the termination solution were added to the blank plate. Therefore, in the samples containing the test compounds or control compounds, the final concentrations of the compounds, testosterone, diclofenac and propafenone in the reaction were 1 μM, the concentration of liver microsomes was 0.5 mg/mL, and the final concentrations of DMSO and acetonitrile in the reaction system were 0.01% (v/v) and 0.99% (v/v), respectively.

After incubating for an appropriate period (*e.g.*, 5, 15, 30, 45 and 60 minutes), 180 μL of termination solution (acetonitrile solution containing 200 ng/mL tolbutamide and 200 ng/mL labetalol) was added to the sample wells of each termination plate, and then 60 μL of sample was removed from the T60 incubation plate to terminate the reaction.

All sample plates were shaken well and centrifuged at 3220 × g for 20 minutes. 80 μL of supernatant from each well was then diluted to 240 μL with pure water for liquid chromatography tandem mass spectrometry analysis. The experimental results are shown in Table 3.

Table 3: Liver microsomal stability test results of the compounds of the present disclosure

| Compound | Human liver microsomal stability $CL_{int}$ (mL/min/kg) |
|---|---|
| Compound **1B** | <15 |
| Compound **3B** | <15 |

Conclusion: The compounds of the present disclosure show good metabolic stability in human liver microsome tests.

**Experimental Example 5: Pharmacokinetic Evaluation in Mice**

The test compound was mixed with 5% DMSO/10% polyethylene glycol 15-hydroxystearate/85% water, vortexed and sonicated to prepare a 0.2 mg/mL clear solution, and then filtered through a microporous membrane for later use. Balb/c male mice weighing 18 to 20 g were selected, and the candidate compound solution was administered intravenously at a dose of 1 mg/kg; the test compound solution was administered orally at a dose of 10 mg/kg. Whole blood was collected for a certain period of time and prepared to obtain plasma. The drug concentration was analyzed by LC-MS/MS method, and the pharmacokinetic parameters were calculated using Phoenix WinNonlin software (Pharsight Company, USA) ($C_0$: concentration needed immediately after intravenous injection; $C_{max}$: the highest plasma drug concentration after administration; $T_{max}$: time needed to reach drug peak concentration after administration; $T_{1/2}$: time needed for plasma drug concentration to decrease by half; $V_{dss}$: apparent volume of distribution, refers to the proportional constant between the amount of drug in the body and the plasma drug concentration when the drug reaches dynamic equilibrium in the body. Cl: clearance rate, refers to the apparent volume of distribution of the drug cleared from the body per unit time; $AUC_{0\text{-}last}$: area under the drug time curve, refers to the area surrounded by the plasma drug concentration

curve against the time axis; F: bioavailability).

The compounds of the present disclosure exhibit a long half-life and high drug exposure with good *in vivo* pharmacokinetic properties.

**Experimental Example 6: Pharmacokinetic Study in Rats**

Male SD rats (6 to 8 weeks old, weighing 200 to 300 g) were selected.

Injection administration (IV) was carried out at a dose of 1 mpk and a concentration of 0.20 mg/mL with a vehicle of 20% PEG400/10% solutol/70% water. Oral administration (PO) was carried out at a dose of 10 mpk and a concentration of 1 mg/mL with a vehicle of 20% PEG400/10% solutol/70% water.

Sample collection: 0.03 mL of blood samples were collected by puncture of the saphenous vein in experimental animals at each time point, and the actual blood collection time was recorded. All blood samples were added to commercial 1.5 mL EDTA-K2 anticoagulant tubes. After blood sample collection, DDV was added into the plasma matrix as a stabilizer, wherein the ratio of plasma to Dichlorvos (DDV) solution was 40:1. The DDV solution was a 40 mM DDV solution in acetonitrile/water (1:1). Within half an hour, the mixture was centrifuged at 4°C and 3000 g for 10 minutes. The supernatant plasma was aspirated, quickly placed in dry ice and stored in a -80°C refrigerator for LC-MS/MS analysis.

A non-compartmental model in Phoenix WinNonlin 6.3 pharmacokinetic software was used to process the plasma concentration data. The pharmacokinetic parameters were calculated using the linear-log trapezoidal method. $C_0$: concentration needed immediately after intravenous injection; $C_{max}$: the highest plasma drug concentration after administration; $T_{max}$: time needed to reach drug peak concentration after administration; $T_{1/2}$: time needed for plasma drug concentration to decrease by half; $V_{dss}$: apparent volume of distribution, refers to the proportional constant between the amount of drug in the body and the plasma drug concentration when the drug reaches dynamic equilibrium in the body. Cl: clearance rate, refers to the apparent volume of distribution of the drug cleared from the body per unit time; $AUC_{0\text{-}last}$: area under the drug time curve, refers to the area surrounded by the plasma drug concentration curve against the time axis; F: bioavailability. The experimental results are shown in Table 4.

Table 4: PK results of compounds of the present disclosure in rats

| **Parameters** | | **$C_0$ (nM)** | **$C_{max}$ (nM)** | **$T_{max}$ (h)** | **$T_{1/2}$ (h)** | **$V_{dss}$ (L/kg)** | **Cl (mL/min/kg)** | **$AUC_{0\text{-}last}$ (nM.h)** | **F (%)** |
|---|---|---|---|---|---|---|---|---|---|
| Compound **2B** | IV | 5813 | -- | -- | 1.16 | 0.55 | 6.5 | 5645 | -- |
| | PO | -- | 9025 | 0.50 | 11.7 | -- | -- | 28425 | 50.4 |
| Compound **3B** | IV | 6200 | -- | -- | 2.06 | 0.53 | 3.5 | 10573 | -- |
| | PO | -- | 7835 | 0.50 | 9.90 | -- | -- | 33029 | 31.2 |
| "--" means that the data were not tested or not obtained. | | | | | | | | | |

Experimental conclusion: The compounds of the present disclosure show a long half-life and high oral plasma exposure, and have good pharmacokinetic properties.

**Experimental Example 7: *In Vitro* Caco-2 Cell Permeability Test**

Cell culture:

Cell culture was performed using MEM (minimum essential medium) supplemented with 2 mM L-glutamine, 10% fetal bovine serum (FBS), 100 U/mL penicillin-G and 100 μg/mL streptomycin. Cell culture conditions were 37 ± 1°C, 5% $CO_2$ and saturated humidity. When the cells reached a density of 80 to 90%, trypsin (0.05%, w/v)/EDTA(0.02%, w/v) digestion solution was added to digest the cells before seeding the plate. Cells were seeded in BD Falcon Transwell-96 well plates at a seeding density of $1 \times 10^5$ cells/cm$^2$. The cells were incubated in a carbon dioxide incubator for 22 days and then used for transport experiments.

Transport experiments:

Hank's balanced salt buffer (pH 7.40 ± 0.05) containing 10 mM HEPES was used as the transport buffer in this project. The bidirectional transport of the test compound and the positive drug digoxin was tested at a concentration of 2 μM, with two parallel samples each, and the transport of fenoterol and propranolol from the apical to the basal end (A-B)

was tested. The concentration of DMSO in the incubation system was controlled to 1% or less. After loading the sample, the cell plate was incubated at 37 ± 1°C, 5% $CO_2$ and saturated humidity for 120 minutes. All samples were vortexed and centrifuged at 3220 rpm and 20°C for 20 minutes. The control sample and test sample were diluted in a 1:1 (v/v) ratio with ultrapure water and stored at 4°C, and the analytical tests were performed by liquid chromatography tandem mass spectrometry (LC/MS/MS).

Sample analysis:

In this study, the liquid chromatography tandem mass spectrometry (LC/MS/MS) method was used to semi-quantitatively analyze the peak area ratios of the test compounds and control samples fenoterol, propranolol and digoxin to the internal standard in the starting solution, receiving solution and administration well supernatant. The apparent permeability coefficient ($P_{app}$, cm/s), efflux rate and recovery rate were calculated using the following formula:

(1) $P_{app} = (dC_r/d_t) \times V_r/(A \times C_0)$, $dC_r/d_t$ is the cumulative concentration of the compound at the receiving end per unit time (μM/s); $V_r$ is the volume of the solution at the receiving end (0.075 mL and 0.250 mL for the solution at the apical and basal ends, respectively); A is the relative surface area of the cell monolayer (0.0804 $cm^2$); $C_0$ is the starting concentration (nM) of the test sample at the administration end or the peak area ratio of the control sample.
(2)

$$\text{Efflux ratio} = P_{app}(BA)/P_{app}(AB)$$

(3) Recovery rate (%) = $100 \times [(V_r \times C_r) + (V_d \times C_d)]/(V_d \times C_0)$, $C_0$ is the starting concentration (nM) of the test sample at the administration end or the peak area ratio of the control sample; $V_d$ is the volume at the administration end (0.075 mL on the apical side and 0.250 mL on the basal side); $C_d$ and $C_r$ are the final concentration (nM) of the test sample or the peak area ratio of the control sample at the administration and receiving ends, respectively.

The experimental results are shown in Table 5.

Table 5. Caco-2 cell permeability test results

| Compound | Apparent permeability coefficient ($10^{-6}$ cm/s) | Efflux ratio |
|---|---|---|
| Compound **1B** | 1.19 | 26.1 |
| Compound **2B** | 1.56 | 15.8 |
| Compound **3B** | 1.21 | 22.6 |

Conclusion: The compounds of the present disclosure have good permeability.

## Claims

**1.** A compound of formula (I) or a pharmaceutically acceptable salt thereof,

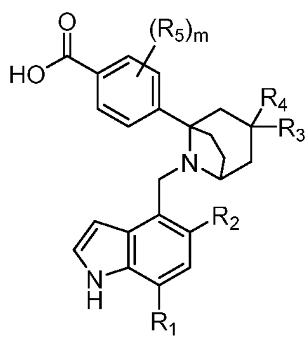

(I) ,

wherein

$R_1$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_a$;

$R_2$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_b$;

$R_3$ is selected from H, D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_c$;

$R_4$ is selected from $C_{1-6}$ alkylthio, $C_{2-6}$ alkenyl, $C_{2-6}$ alkynyl, $C_{3-6}$ cycloalkyl and 3- to 6-membered heterocyclyl, and the $C_{1-6}$ alkylthio, $C_{2-6}$ alkenyl, $C_{2-6}$ alkynyl, $C_{3-6}$ cycloalkyl and 3- to 6-membered heterocyclyl are each independently and optionally substituted by 1, 2, 3 or 4 $R_d$;

each $R_5$ is independently selected from D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 $R_e$;

each $R_a$ is independently selected from D, F, Cl, Br and I;

each $R_b$ is independently selected from D, F, Cl, Br and I;

each $R_c$ is independently selected from D, F, Cl, Br and I;

each $R_d$ is independently selected from D, F, Cl, Br, I, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 R;

each $R_e$ is independently selected from D, F, Cl, Br and I;

each R is independently selected from D, F, Cl, Br and I;

m is selected from 0, 1, 2 and 3.

**2.** The compound or the pharmaceutically acceptable salt thereof according to claim 1, wherein each $R_d$ is independently selected from D, F, Cl, CN, OH, $NH_2$, $C_{1-3}$ alkyl and $C_{1-3}$ alkoxy, and the $C_{1-3}$ alkyl and $C_{1-3}$ alkoxy are each independently and optionally substituted by 1, 2 or 3 R; or, each $R_d$ is independently selected from F and $C_{1-3}$ alkyl, and the $C_{1-3}$ alkyl is optionally substituted by 1, 2 or 3 R; or, each $R_d$ is independently selected from F and methyl.

**3.** The compound or the pharmaceutically acceptable salt thereof according to claim 1, wherein $R_1$ is selected from $C_{1-3}$ alkyl, and the $C_{1-3}$ alkyl is optionally substituted by 1, 2 or 3 $R_a$; or, $R_1$ is selected from $CH_3$, and the $CH_3$ is optionally substituted by 1, 2 or 3 $R_a$; or, $R_1$ is selected from $CH_3$ and $CD_3$.

**4.** The compound or the pharmaceutically acceptable salt thereof according to claim 1, wherein $R_2$ is selected from $C_{1-3}$ alkoxy, and the $C_{1-3}$ alkoxy is optionally substituted by 1, 2 or 3 $R_b$; or, $R_2$ is selected from $OCH_3$, and the $OCH_3$ is optionally substituted by 1, 2 or 3 $R_b$; or, $R_2$ is selected from $OCH_3$ and $OCD_3$.

**5.** The compound or the pharmaceutically acceptable salt thereof according to claim 1, wherein $R_3$ is selected from H, F and $C_{1-3}$ alkyl, and the $C_{1-3}$ alkyl is optionally substituted by 1, 2 or 3 $R_c$; or, $R_3$ is selected from H, F and $CH_3$; or, $R_3$ is selected from H.

**6.** The compound or the pharmaceutically acceptable salt thereof according to claim 1, wherein $R_4$ is selected from $C_{1-6}$ alkylthio, $C_{2-6}$ alkenyl, $C_{2-6}$ alkynyl and $C_{3-6}$ cycloalkyl, and the $C_{1-6}$ alkylthio, $C_{2-6}$ alkenyl, $C_{2-6}$ alkynyl and $C_{3-6}$ cycloalkyl are each independently and optionally substituted by 1, 2, 3 or 4 $R_d$; or, $R_4$ is selected from $C_{1-3}$ alkylthio, $C_{2-3}$ alkenyl, $C_{2-3}$ alkynyl and $C_{3-4}$ cycloalkyl, and the $C_{1-3}$ alkylthio, $C_{2-3}$ alkenyl, $C_{2-3}$ alkynyl and $C_{3-4}$ cycloalkyl are each independently and optionally substituted by 1, 2, 3 or 4 $R_d$.

**7.** The compound or the pharmaceutically acceptable salt thereof according to claim 6, wherein $R_4$ is selected from

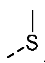, 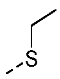, 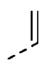, 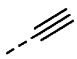, 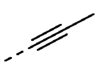, 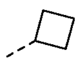 and 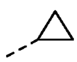,

and the

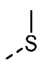,

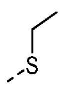, , 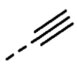, 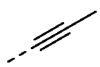, 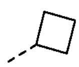 and 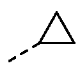

are each independently and optionally substituted by 1, 2, 3 or 4 $R_d$; or, $R_4$ is selected from

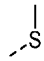, 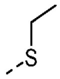, 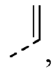, 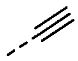 and 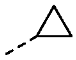,

and

the 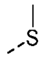, 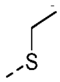, 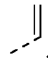, 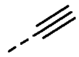 and 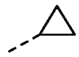

are each independently and optionally substituted by 1, 2, 3 or 4 $R_d$; or, $R_4$ is selected from

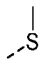, 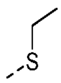, 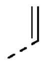, 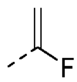, 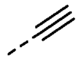, 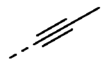, 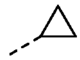, 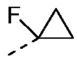,

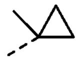, 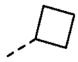 and 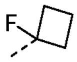;

or, $R_4$ is selected from

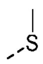, 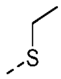, 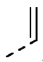, 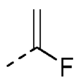, 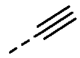 and 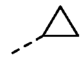.

**8.** The compound or the pharmaceutically acceptable salt thereof according to claim 1, wherein m is selected from 0.

**9.** The compound or the pharmaceutically acceptable salt thereof according to claim 1, each $R_a$, each $R_b$, each $R_c$, each $R_e$ and each R are independently selected from D and F.

**10.** The compound or the pharmaceutically acceptable salt thereof according to any one of claims 1 to 9, selected from a compound of formula (P), a compound of formula (P-1) or a compound of formula (P-2),

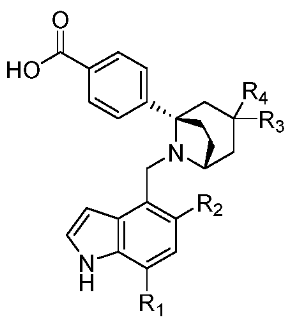
(P),

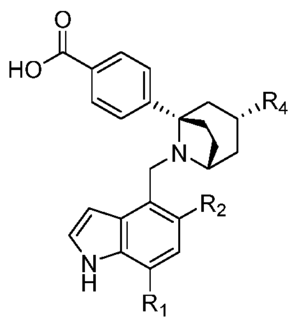
(P-1),

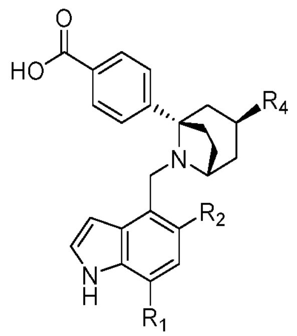
(P-2),

wherein $R_1$, $R_2$, $R_3$ and $R_4$ are as defined in any one of claims 1 to 9.

**11.** The compound or the pharmaceutically acceptable salt thereof according to claim 10, wherein

$R_1$ is selected from $C_{1-3}$ alkyl, and the $C_{1-3}$ alkyl is optionally substituted by 1, 2 or 3 $R_a$;
$R_2$ is selected from $C_{1-3}$ alkoxy, and the $C_{1-3}$ alkoxy is optionally substituted by 1, 2 or 3 $R_b$;
$R_3$ is selected from H;
$R_4$ is selected from $C_{1-3}$ alkylthio, $C_{2-3}$ alkenyl, $C_{2-3}$ alkynyl and $C_{3-4}$ cycloalkyl, and the $C_{1-3}$ alkylthio, $C_{2-3}$ alkenyl,

$C_{2-3}$ alkynyl and $C_{3-4}$ cycloalkyl are each independently and optionally substituted by 1, 2, 3 or 4 $R_d$;
each $R_a$ is independently selected from D, F, Cl, Br and I;
each $R_b$ is independently selected from D, F, Cl, Br and I;
each $R_d$ is independently selected from D, F, Cl, Br, I, $C_{1-3}$ alkyl and $C_{1-3}$ alkoxy, and the $C_{1-3}$ alkyl and $C_{1-3}$ alkoxy are each independently and optionally substituted by 1, 2 or 3 R;
each R is independently selected from D, F, Cl, Br and I.

**12.** A compound as shown below or a pharmaceutically acceptable salt thereof,

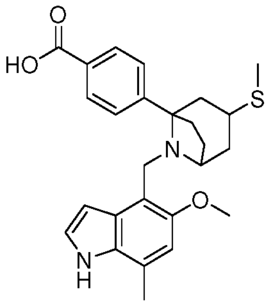 , 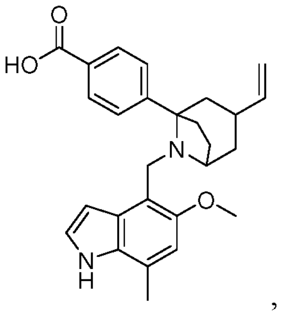 , 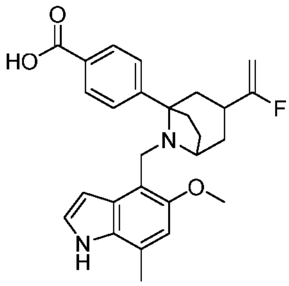 , 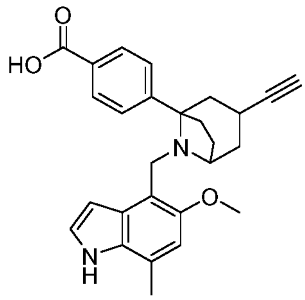 ,

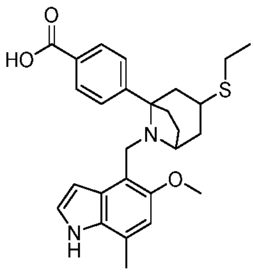 , 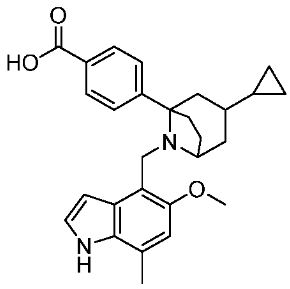 , 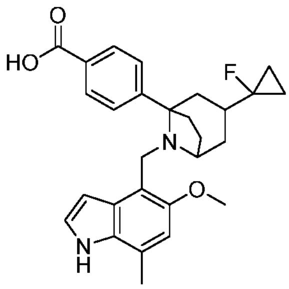 , 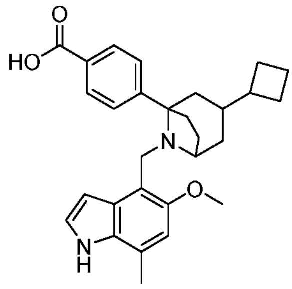 ,

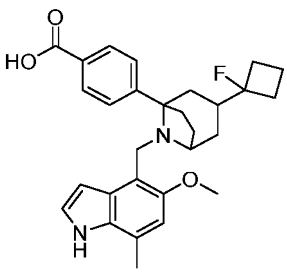 , 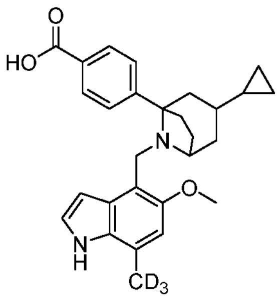 , 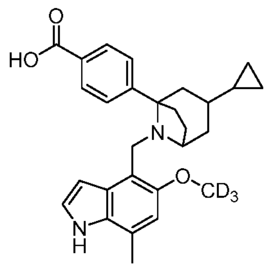 , 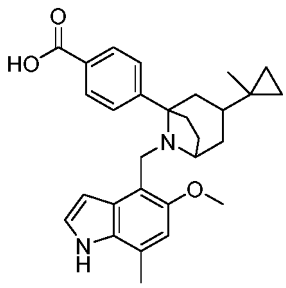 ,

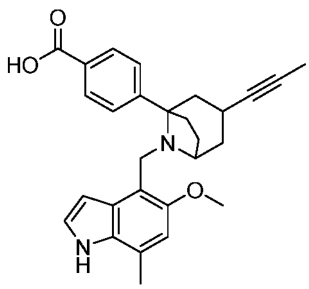 , 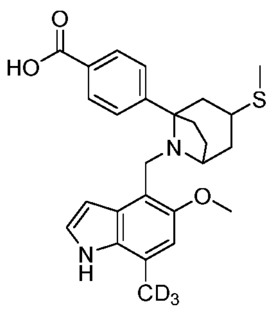 , 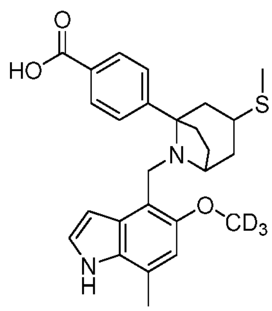

and

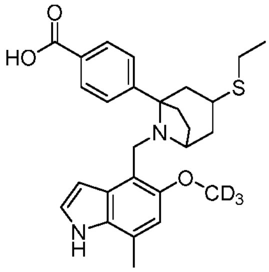

.

**13.** The compound or the pharmaceutically acceptable salt thereof according to claim 12, wherein the compound is selected from:

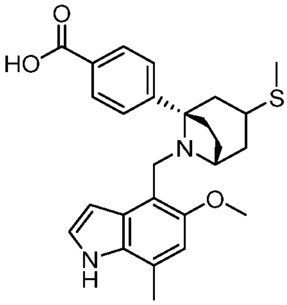
,
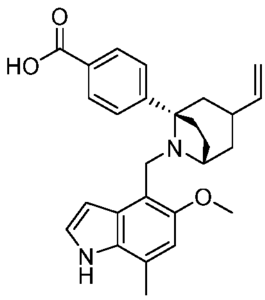
,
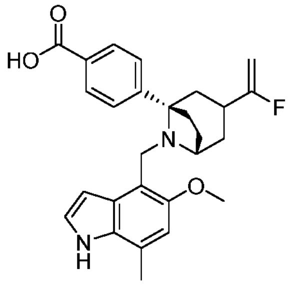
,
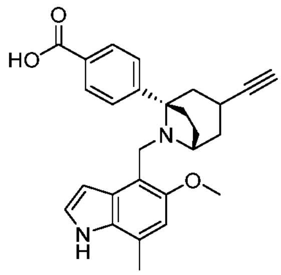
,
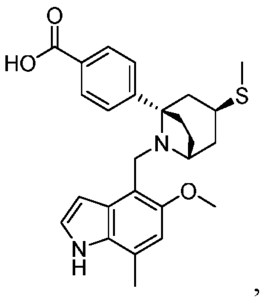
,
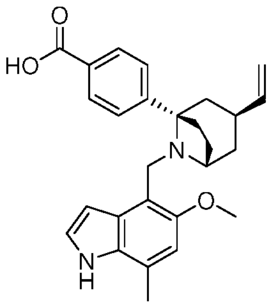
,
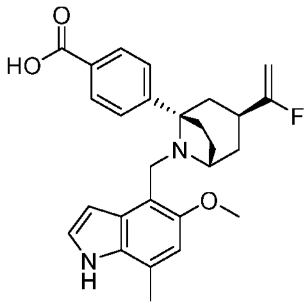
,
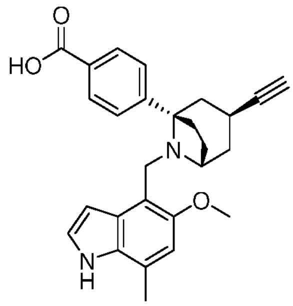
,
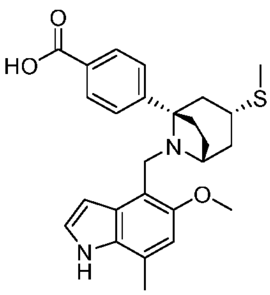
,
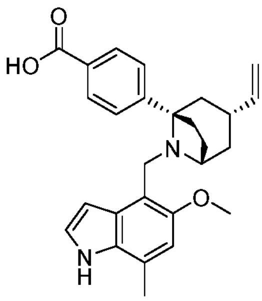
,
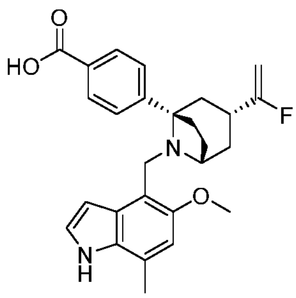
,
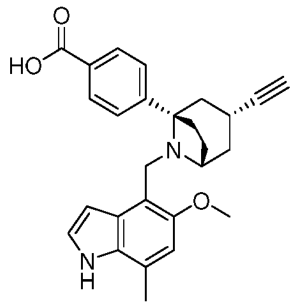
,
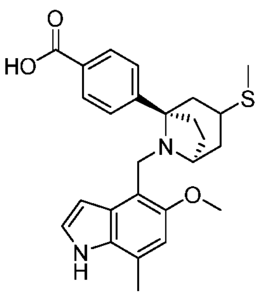
,
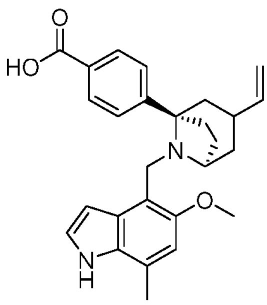
,
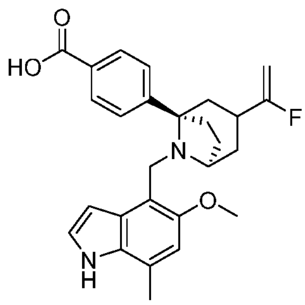
,
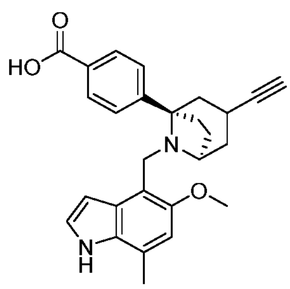
,

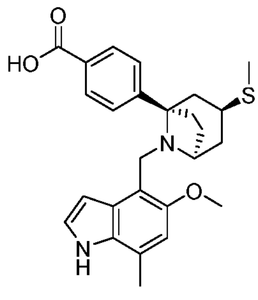 , 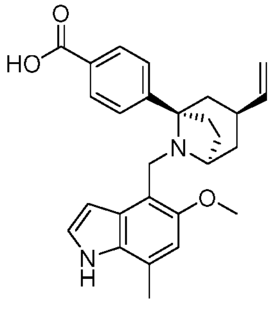 , 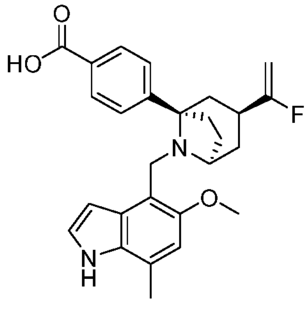 , 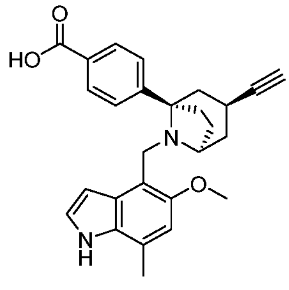 ,

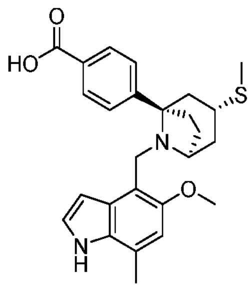 , 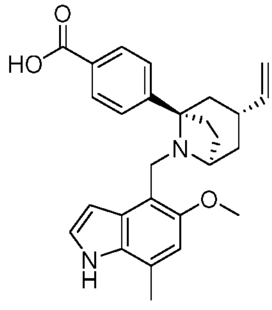 , 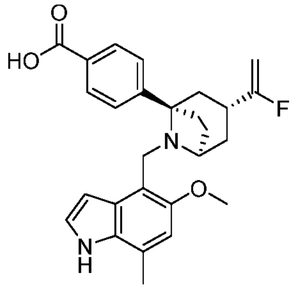 , 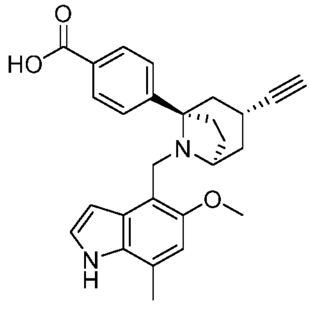 ,

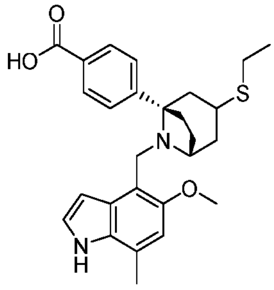 , 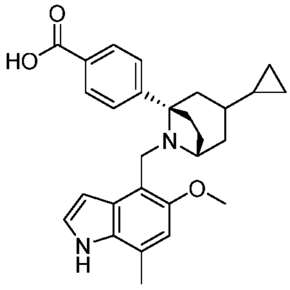 , 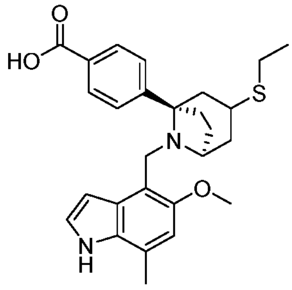 , 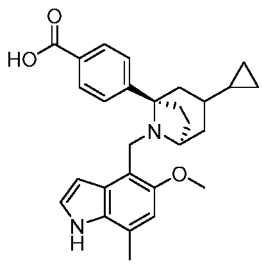 ,

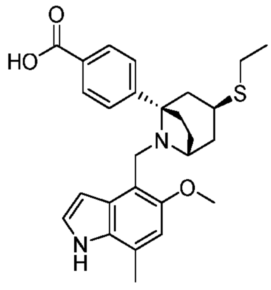 , 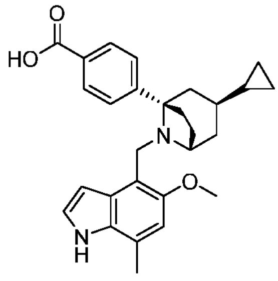 , 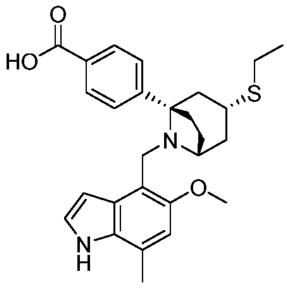 , 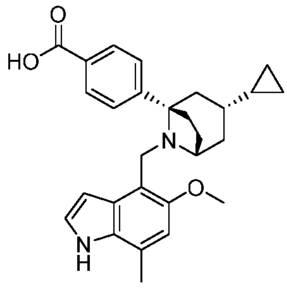 ,

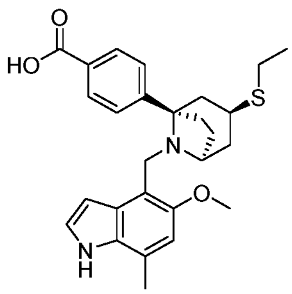 , 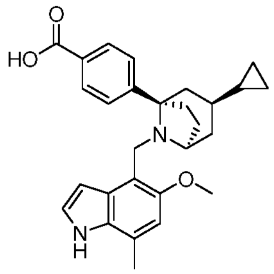 , 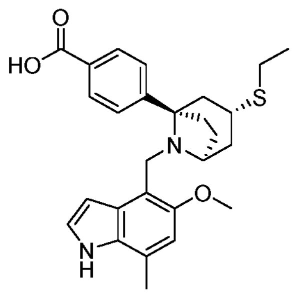 , 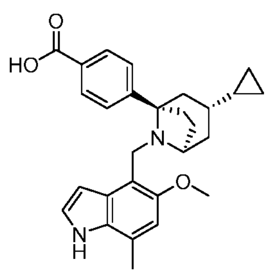 ,

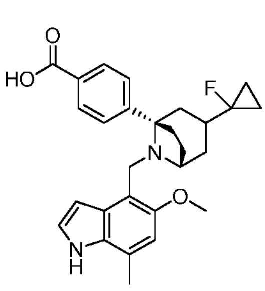,
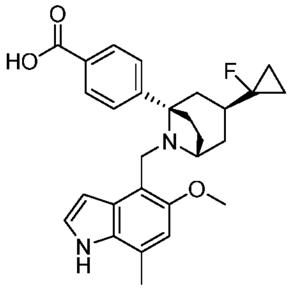,
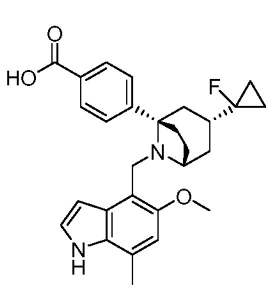,
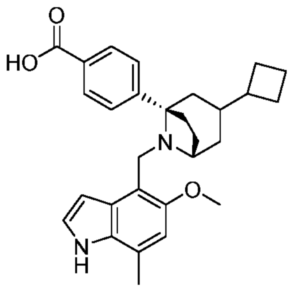,

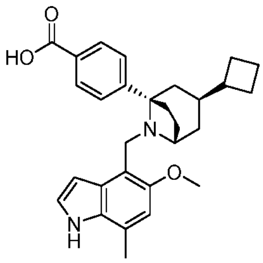,
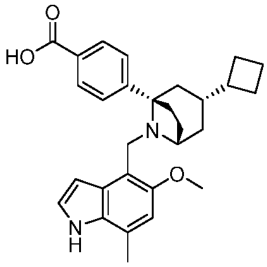,
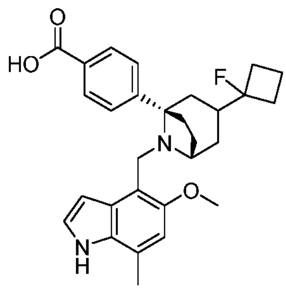,
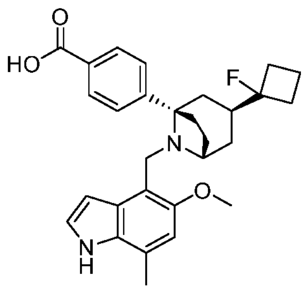,

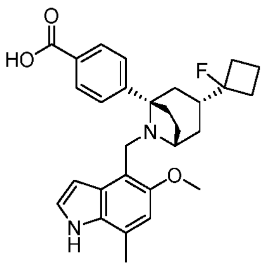,
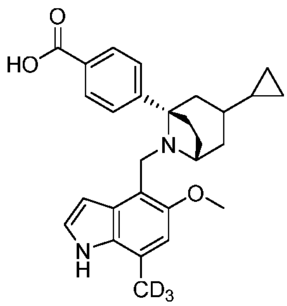,
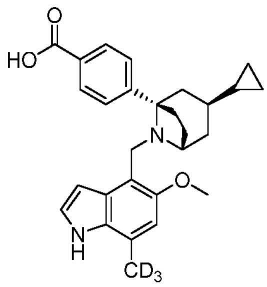,
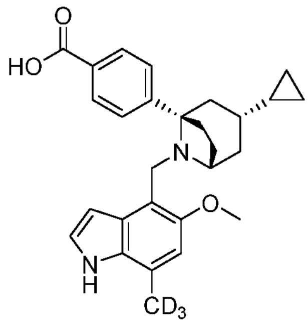,

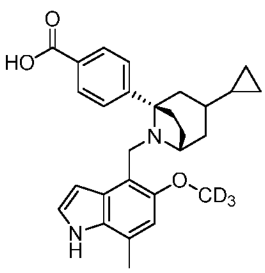,
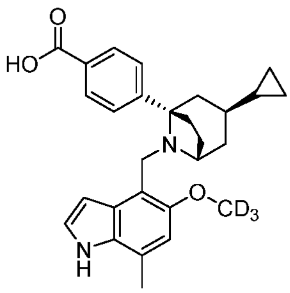,
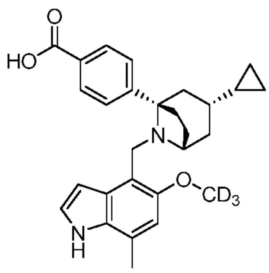,
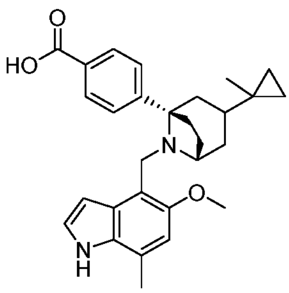,

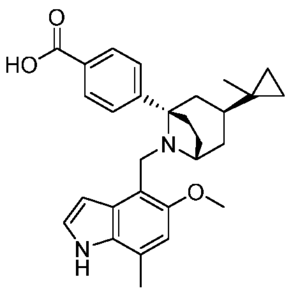,
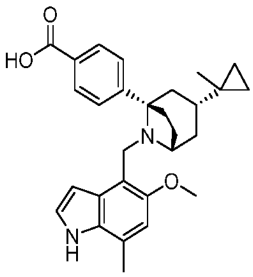,
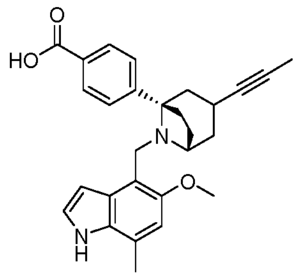,
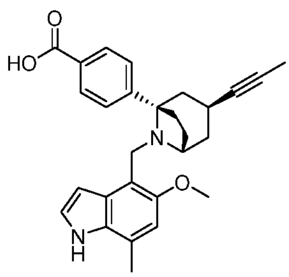,

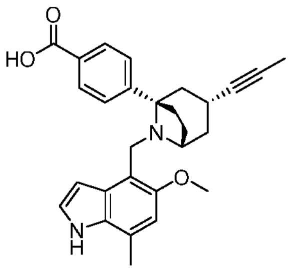, 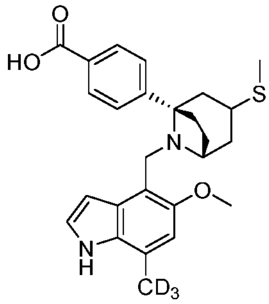, 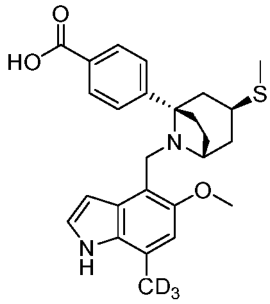, 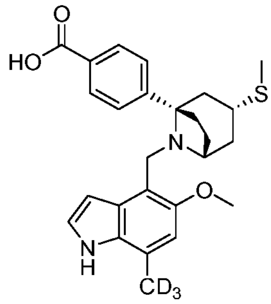,

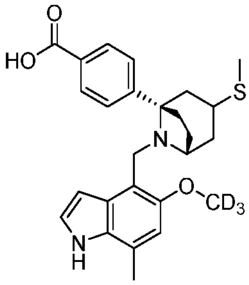, 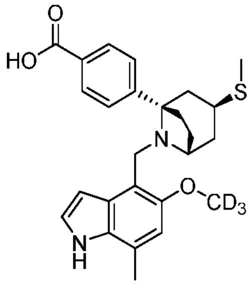, 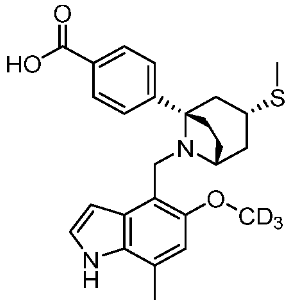, 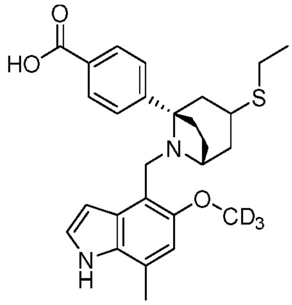,

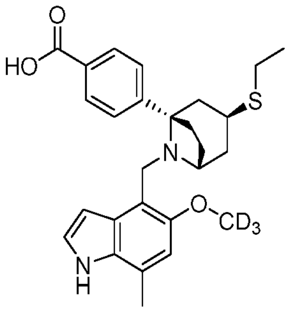 and 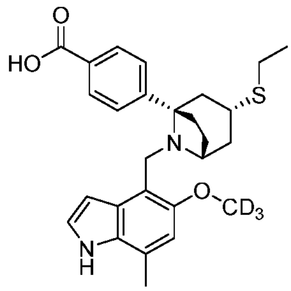.

**14.** A pharmaceutical composition comprising a therapeutically effective amount of the compound or the pharmaceutically acceptable salt thereof according to any one of claims 1 to 13, optionally further comprising a pharmaceutically acceptable carrier.

**15.** Use of the compound or the pharmaceutically acceptable salt thereof according to any one of claims 1 to 13, or the pharmaceutical composition according to claim 14 in the manufacture of a medicament for treating diseases associated with complement Factor B.

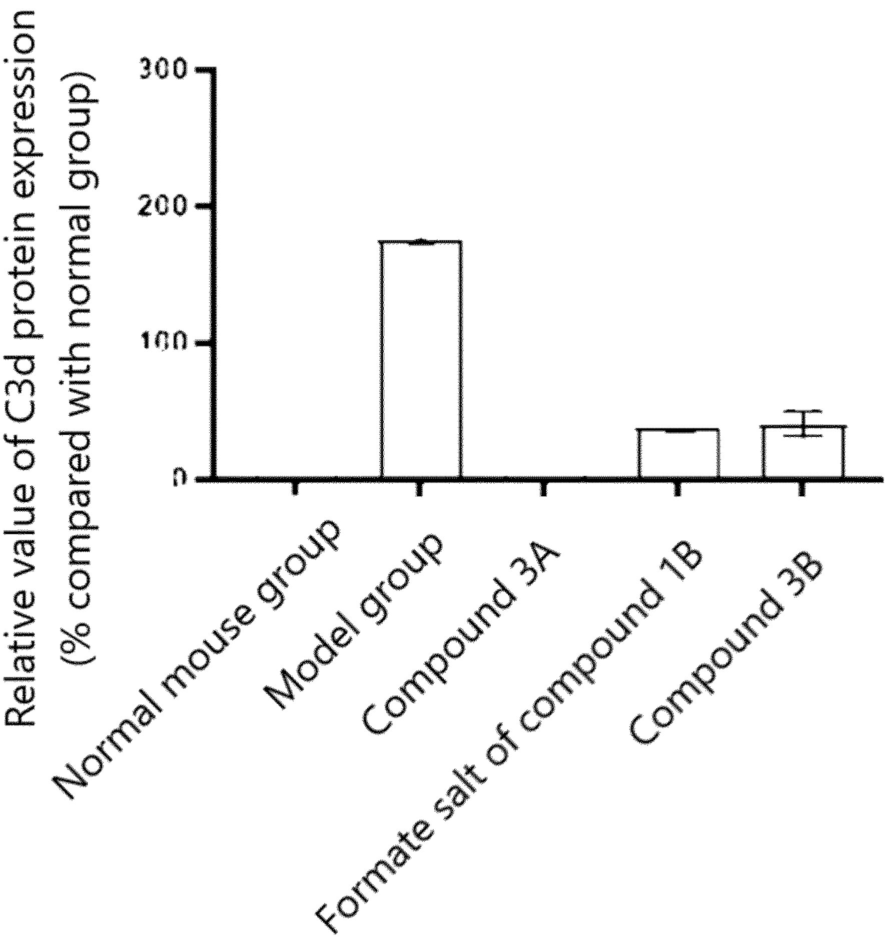
Relative value of C3d protein expression
(% compared with normal group)
300
200
100
0
Normal mouse group
Model group
Compound 3A
Formate salt of compound 1B
Compound 3B

FIG. 1

**INTERNATIONAL SEARCH REPORT**

International application No. **PCT/CN2023/115288**

**A. CLASSIFICATION OF SUBJECT MATTER**

C07D401/02(2006.01)i; C07D401/06(2006.01)i; C07D471/08(2006.01)i; C07D451/06(2006.01)i; C07D495/04(2006.01)i; C07D491/06(2006.01)i; C07D471/04(2006.01)i; A61K31/439(2006.01)i; A61K31/706(2006.01)i; A61P9/00(2006.01)i; A61P13/12(2006.01)i; A61P25/00(2006.01)i; A61P27/02(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNKI, VCN, VEN, DWPI, CNTXT, ENTXT, ENTXTC, STN (REG, CAPLUS), 补体因子B抑制剂, 氮桥杂环, Complement factor B inhibitor, Nitrogen-containing bridge heterocyclic, Factor B, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 114057758 A (SHANGHAI MEIYUE BIOTECHNOLOGY DEVELOPMENT CO., LTD.) 18 February 2022 (2022-02-18)<br>claims 1, 3, 5, and 8-10, and description, page 99 | 1-15 |
| PX | CN 116438170 A (JIANGSU HENGRUI MEDICINE CO., LTD. et al.) 14 July 2023 (2023-07-14)<br>claims 1-21 | 1-15 |
| PX | WO 2022218429 A1 (CHIA TAI TIANQING PHARMACEUTICAL GROUP CO., LTD. et al.) 20 October 2022 (2022-10-20)<br>claims 1-17 | 1-15 |
| PA | WO 2023020566 A1 (SICHUAN HAISCO PHARMACEUTICAL CO., LTD.) 23 February 2023 (2023-02-23)<br>entire document | 1-15 |
| A | CN 105579444 A (NOVARTIS AG) 11 May 2016 (2016-05-11)<br>entire document | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 December 2023** | **12 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/115288**

| Patent document cited in search report | Publication date (day/month/year) | Patent family member(s) | Publication date (day/month/year) |
|---|---|---|---|
| CN 114057758 A | 18 February 2022 | US 2023286947 A1 | 14 September 2023 |
| | | AU 2021323300 A1 | 06 April 2023 |
| | | KR 20230049115 A | 12 April 2023 |
| | | BR 112023001195 A2 | 28 February 2023 |
| | | JP 2023538844 A | 12 September 2023 |
| | | IL 300432 A | 01 April 2023 |
| | | CA 3188363 A1 | 10 February 2022 |
| | | EP 4194451 A1 | 14 June 2023 |
| | | WO 2022028527 A1 | 10 February 2022 |
| | | TW 202214653 A | 16 April 2022 |
| | | TWI 811756 B | 11 August 2023 |
| CN 116438170 A | 14 July 2023 | CA 3203320 A1 | 07 July 2022 |
| | | KR 20230128039 A | 01 September 2023 |
| | | TW 202229271 A | 01 August 2022 |
| | | AU 2021414185 A1 | 10 August 2023 |
| | | WO 2022143845 A1 | 07 July 2022 |
| | | EP 4273136 A1 | 08 November 2023 |
| WO 2022218429 A1 | 20 October 2022 | CN 117062810 A | 14 November 2023 |
| | | CA 3214014 A1 | 20 October 2022 |
| | | AU 2022259328 A1 | 09 November 2023 |
| WO 2023020566 A1 | 23 February 2023 | TW 202321212 A | 01 June 2023 |
| CN 105579444 A | 11 May 2016 | IL 243540 A0 | 29 February 2016 |
| | | IL 243540 B | 26 September 2019 |
| | | PL 3022192 T3 | 30 March 2018 |
| | | DK 3022192 T3 | 22 January 2018 |
| | | MY 187454 A | 22 September 2021 |
| | | UA 117371 C2 | 25 July 2018 |
| | | JP 2016526576 A | 05 September 2016 |
| | | JP 6378761 B2 | 22 August 2018 |
| | | NZ 715780 A | 26 March 2021 |
| | | AP 201608992 D0 | 31 January 2016 |
| | | EA 201690223 A1 | 30 June 2016 |
| | | EA 031030 B1 | 30 November 2018 |
| | | AP 2016008992 A0 | 31 January 2016 |
| | | PH 12016500072 A1 | 18 April 2016 |
| | | JO 3425 B1 | 20 October 2019 |
| | | CU 20160006 A7 | 31 August 2016 |
| | | CU 24397 B1 | 04 April 2019 |
| | | PE 20161066 A1 | 30 October 2016 |
| | | US 2018009795 A1 | 11 January 2018 |
| | | US 10093663 B2 | 09 October 2018 |
| | | AU 2014290298 A1 | 11 February 2016 |
| | | AU 2014290298 B2 | 15 June 2017 |
| | | MX 351291 B | 09 October 2017 |
| | | SG 11201600086 PA | 26 February 2016 |
| | | HRP 20172000 T1 | 09 February 2018 |
| | | CA 2917839 A1 | 22 January 2015 |
| | | CA 2917839 C | 03 May 2022 |
| | | PT 3022192 T | 15 January 2018 |
| | | TW 201546058 A | 16 December 2015 |

Form PCT/ISA/210 (patent family annex) (July 2022)

**INTERNATIONAL SEARCH REPORT**
**Information on patent family members**

International application No.
**PCT/CN2023/115288**

| Patent document cited in search report | Publication date (day/month/year) | Patent family member(s) | | | Publication date (day/month/year) |
|---|---|---|---|---|---|
| | | TWI | 641600 | B | 21 November 2018 |
| | | CY | 1119767 | T1 | 27 June 2018 |
| | | HK | 1221217 | A1 | 26 May 2017 |
| | | LT | 3022192 | T | 27 December 2017 |
| | | SI | 3022192 | T1 | 31 January 2018 |
| | | UY | 35663 | A | 27 February 2015 |
| | | IL | 268623 | A | 31 October 2019 |
| | | IL | 268623 | B | 30 January 2020 |
| | | GT | 201600007 | A | 18 December 2018 |
| | | EP | 3299365 | A1 | 28 March 2018 |
| | | HUE | 037510 | T2 | 28 September 2018 |
| | | CL | 2016000060 | A1 | 10 June 2016 |
| | | WO | 2015009616 | A1 | 22 January 2015 |
| | | MY | 196427 | A | 10 April 2023 |
| | | TN | 2016000017 | A1 | 05 July 2017 |
| | | SV | 2016005137 | A | 12 June 2018 |
| | | BR | 112016000909 | A2 | 25 July 2017 |
| | | BR | 112016000909 | A8 | 07 January 2020 |
| | | BR | 112016000909 | B1 | 02 May 2023 |
| | | ES | 2655855 | T3 | 21 February 2018 |
| | | US | 2016152605 | A1 | 02 June 2016 |
| | | US | 9682968 | B2 | 20 June 2017 |
| | | KR | 20160030556 | A | 18 March 2016 |
| | | KR | 102242742 | B1 | 23 April 2021 |
| | | EP | 3022192 | A1 | 25 May 2016 |
| | | EP | 3022192 | B1 | 11 October 2017 |
| | | RS | 56721 | B1 | 30 March 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2022110640297
- CN 2022112310018
- CN 2023110663471